# EUROPEAN PATENT APPLICATION

(11) **EP 4 393 910 A1**
(43) Date of publication of application: **03.07.2024**
(21) Application number: 22217081.3
(22) Date of filing: 29.12.2022
(51) Int. Cl.: C07D 251/18, C07C 279/00

(54) **PROCESS FOR THE PREPARATION OF BIGUANIDINE AND TRIAZINES**

(71) Applicant: Adama Agan Ltd., 7710001 Ashdod (IL)
(72) Inventor: SIMAAN, Marwan E, 1623403 Nazareth (IL); ERCHOV, Leonid, 9902004 Beit Shemesh (IL)
(74) Representative: Modiano, Gabriella Diana

(57) **Abstract**

A process for the preparation of biguanidine compounds of formula (I) and triazine compounds of formula (IV) 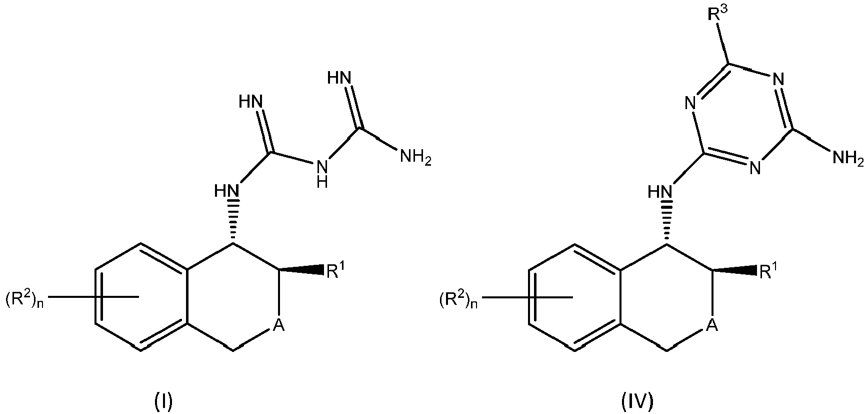 wherein R¹, R² and R³ are each independently hydrogen or an optionally substituted C₁-C₄ alkyl group, wherein the optional substituents are selected from the group consisting of halogen, aliphatic, halo aliphatic, alicyclic, alkoxy, thioalkyl, cyano or nitro groups; A is -CH₂-, -O- or a direct bond; and n is 0, 1, 2 or 3.

## Description

### FIELD OF THE INVENTION

The present invention belongs to the field of agrochemistry. It is directed to a process for preparation of biguanidines and triazines, wherein the triazines are suitable as herbicides for controlling weeds.

### BACKGROUND

Triazine compounds are a class of compounds suitable for being used as herbicides. Triazine compounds such as atrazine, ametryne, indaziflam or triaziflam are among the compounds that are used as herbicides

The prior art discloses in EP1592674 A1, EP2231679 A1 or EP3347342 A1 different methods for preparing indaziflam. One of the methods disclosed in the prior art document EP1592674 A1 relates to the preparation of biguanidine intermediates of formula (I): 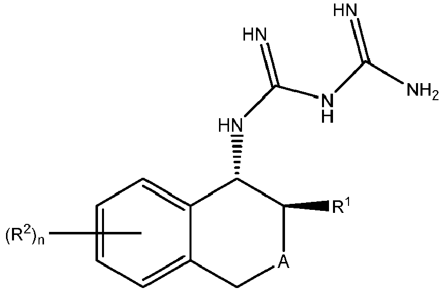 from the reaction of 1-cyanoguanidine with an amine of formula (II): 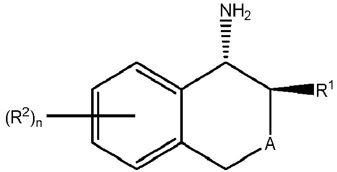 in a solvent such as 1,2-dichlorobenzene, decalin or white mineral oil, at a temperature of from 20ºC to the reflux temperature of the solvent, preferably at 50ºC to 200ºC.

According to that document, the biguanidine intermediate of formula (I) is obtained with a yield of 67.7% when the reaction of (1*R*,2*S*)-1-amino-2-methylindane hydrochloride and 1-cyanoguanidine is performed in 1,3-dichlorobenzene at 140-150 ºC.

Also according to this document EP1592674 A1, the biguanidine intermediate of formula (I) or an acid addition salt thereof reacts with a carboxylic acid derivative of formula (III): Z-R³, in the presence of a base, in an inert solvent such as e.g. a polar organic solvent such as tetrahydrofuran, dioxan, acetonitrile, N,N-dimethylformamide, methanol or ethanol, at a temperature of from 0 ºC to the reflux temperature of the solvent, preferably at 20 ºC to 100 ºC, to yield a triazine compound of formula (IV):

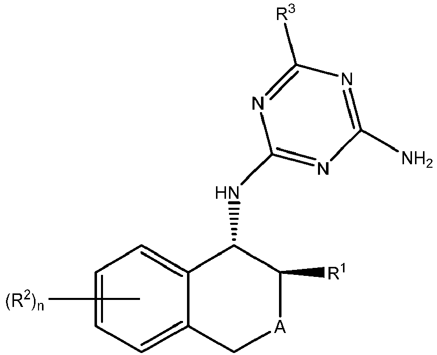

This synthesis of compounds of formula (IV) according to the herein disclosed synthesis in EP1592674 A1 shows the drawback of the low yield in the preparation of biguanidine intermediate of formula (I) or an acid addition salt thereof.

Additional approach described in the prior art is described in EP2231679 A1. That document refers to the use of aluminum alkoxides as an additive in a first step to form a biguanidino-aluminum complex. The addition of an aluminum alkoxide allows the first reaction to progress under low temperature and form a stable biguanidino-aluminum complex intermediate which also reacts in a "one-pot" fashion to afford the final indaziflam with better yield than the yield reported in EP1592674 A1. However, the approach in this prior art document requires the use of large excess of aluminum alkoxide, which results later on in considerable amounts of waste which requires costly and complex disposal and it is a major drawback in industrial production. The process also requires a combination of several different solvents for each step, which is an operational challenge while trying to recycle and isolate each of them.

The third method for preparing indaziflam know in the prior art is disclosed in EP3347342 A1. According to that document, the hereinabove disclosed preparation of indaziflam according to EP1592674 A1 is achieved with higher yield than in EP1592674 A1 when an autocatalytic amount of the biguanidine intermediate of formula (I) is added to the preparation of the biguanidine intermediate of formula (I) and the reaction is preferably performed at a temperature from 140ºC to 148ºC. The process also offers a "one-pot" synthesis, while the second step from the biguanidine intermediate of formula (I) to indaziflam involves the addition of a phase transfer catalyst and potassium carbonate as a base. However, the first step is still performed under very high temperatures and the process also requires the filtration of the excess of potassium carbonate salt, which makes the isolation more complicated operationally.

Therefore, the present invention provides an alternative to the existing methods for the preparation of indaziflam and it is an object of the present invention to provide a process which solves some of the drawbacks of low yield, considerable amounts of waste to be handled, high temperature of the reaction or operationally complicated isolation.

### SUMMARY OF THE INVENTION

A first aspect of the invention is a process for the preparation of a biguanidine compound of formula (I) or an acid addition salt thereof: 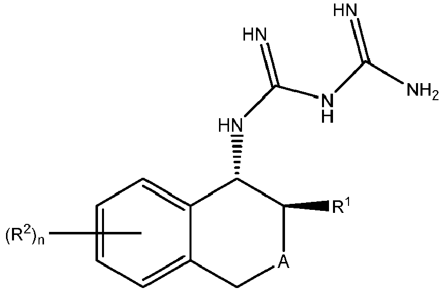
wherein R¹ and R² are each independently hydrogen or an optionally substituted C₁-C₄ alkyl group, wherein the optional substituents are selected from the group consisting of halogen, aliphatic, halo aliphatic, alicyclic, alkoxy, thioalkyl, cyano or nitro groups; A is -CH₂-, -O- or a direct bond; and n is 0, 1, 2 or 3;
which comprises the reaction of 1-cyanoguanidine with an amine of formula (II) or an acid addition salt thereof 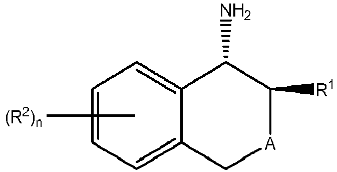
wherein R¹, R², A and n are as defined in formula (I), in a mixture which comprises a trimethylsilyl-containing compound, and an optionally alkyl substituted N-alkyl γ-, δ-, ε-lactam or an optionally alkyl substituted γ-, δ-, ε-lactone.

A second aspect of the invention is a process for the preparation of a triazine compound of formula (IV): 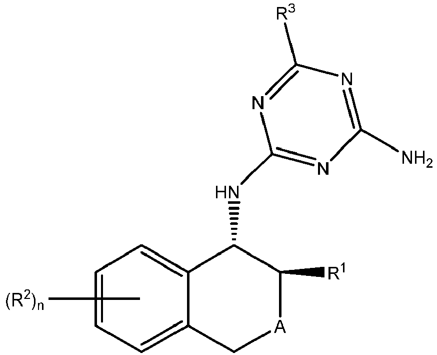 wherein R¹, R² and R³ are each independently hydrogen or an optionally substituted C₁-C₄ alkyl group, wherein the optional substituents are selected from the group consisting of halogen, aliphatic, halo aliphatic, alicyclic, alkoxy, thioalkyl, cyano or nitro groups; A is -CH₂-, -O- or a direct bond; and n is 0, 1, 2 or 3; which comprises
a) a first step of preparation of a biguanidine compound of formula (I) or an acid addition salt thereof: 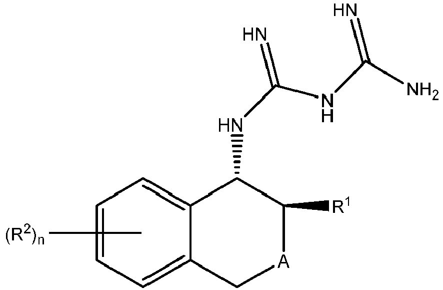
   wherein R¹, R², A and n are as defined in formula (IV);
   by reaction of 1-cyanoguanidine with an amine of formula (II) or an acid addition salt thereof 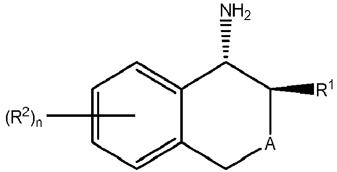
   wherein R¹, R², A and n are as defined in formula (IV), in a mixture which comprises a trimethylsilyl-containing compound, and an optionally alkyl substituted N-alkyl γ-, δ-, ε-lactam or an optionally alkyl substituted γ-, δ-, ε-lactone;
b) a base and a carboxylic acid derivative of formula (III) are added to the biguanidine compound of formula (I) or an acid addition salt thereof, obtained in step a)

   Z-R³ (III)

   wherein R³ is as defined in formula (IV), and Z-R³ is selected from the group consisting of carboxylic acid esters, carboxylic orthoesters, carboxylic acid chlorides, carboxamides, nitriles, or carboxylic anhydrides

### DETAILED DESCRIPTION OF THE INVENTION

### Definitions

Embodiments of the present invention are discussed in detail below. In describing embodiments, specific terminology is employed for the sake of clarity. However, the invention is not intended to be limited to the specific terminology so selected. While specific exemplary embodiments are discussed, it should be understood that this is done for illustration purposes only. A person skilled in the relevant art will recognize that other components and configurations can be used without parting from the spirit and scope of the invention. While a number of embodiments and features are described herein, it is to be understood that the various features of the invention and aspects of embodiments, even if described separately, may be combined unless mutually exclusive or contrary to the specific description. All references cited herein are incorporated by reference as if each had been individually incorporated.

As used herein, the transitional term "comprising" or "that comprises", which is synonymous with "including," or "containing," is inclusive or open-ended and does not exclude additional, un-recited elements or method steps. However, in each recitation of "comprising" herein, it is intended that the term also encompass, as alternative embodiments, the phrases "consisting essentially of" and "consisting of", where "consisting of" excludes any element or step not specified and "consisting essentially of" permits the inclusion of additional un-recited elements or steps that do not materially affect the essential or basic and novel characteristics of the composition or method under consideration.

Prior to setting forth the present subject matter in detail, it may be helpful to provide definitions of certain terms to be used herein. Unless defined otherwise, all technical and scientific terms used herein have the same meaning as is commonly understood by one of skill in the art to which this subject matter pertains.

The term "optionally alkyl substituted N-alkyl γ-, δ-, ε-lactam" means a lactam with a 5-, 6-, or 7-membered cycle, respectively, wherein the nitrogen atom is substituted with an alkyl group, and optionally one or several carbon atoms of the cycle are substituted with one or several alkyl groups.

The term "optionally alkyl substituted γ-, δ-, ε-lactone" means a lactone with a 5-, 6-, or 7-membered cycle, respectively, wherein optionally one or several carbon atoms of the cycle are substituted with one or several alkyl groups.

The term "trimethylsilyl-containing compound" refers to a compound that contains at least one trimethylsilyl group within its molecule.

The term "alkoxy group" refers to an alkyl group bound to oxygen.

The term "thioalkyl group" refers to an alkyl group bound to sulfur.

The term "halo aliphatic group" refers to an aliphatic group substituted with at least one halogen atom.

The term "aliphatic group" is used in this invention to cover, for example and not restricted to, the linear or branched alkyl, alkenyl and alkynyl groups.

The term "alkyl group" refers to a saturated, linear or branched group, which has between 1 and 24, between 1 and 16, between 1 and 14, between 1 and 12, 1, 2, 3, 4, 5 or 6 carbon atoms and is bound to the rest of the molecule by a single bond, including, for example and not restricted to, methyl, ethyl, isopropyl, isobutyl, tert-butyl, heptyl, octyl, decyl, dodecyl, hexadecyl, octadecyl, amyl, 2-ethylhexyl, 2-methylbutyl, 5-methylhexyl and similar.

The term "halo alkyl group" refers to an alkyl group substituted with at least one halogen atom.

The term "alkenyl group" refers to a linear or branched group, which has between 2 and 24, between 2 and 16, between 2 and 14, between 2 and 12, 2, 3, 4, 5 or 6 carbon atoms, with 1, 2 or 3 carbon-carbon double bonds, conjugated or unconjugated, which is bound to the rest of the molecule by a single bond, including, for example and not restricted to, vinyl, allyl, oleyl, linoleyl and similar groups.

The term "alkynyl group" refers to a linear or branched group, which has between 2 and 24, between 2 and 16, between 2 and 14, between 2 and 12, 2, 3, 4, 5 or 6 carbon atoms, with 1, 2 or 3 carbon-carbon triple bonds, conjugated or unconjugated, which is bound to the rest of the molecule by a single bond, including, for example and not restricted to, the ethynyl group, 1-propinyl, 2-propinyl, 1-butinyl, 2-butinyl, 3-butinyl, pentinyl, such as 1-pentinyl, and similar.

The term "alkylidene group" refers to a saturated, linear or branched group, which has between 1 and 24, between 1 and 16, between 1 and 14, between 1 and 12, 1, 2, 3, 4, 5 or 6 carbon atoms and is bound to the rest of the molecule by a double bond, including, for example and not restricted to, methylidene, ethylidene, isopropylidene, isobutylidene, tert-butylidene, heptylidene, octylidene, decylidene, dodecylidene, hexadecylidene and similar.

The term "alycyclic group" is used in this invention to cover, for example and not restricted to, cycloalkyl or cycloalkenyl or cycloalkynyl groups.

The term "cycloalkyl" refers to a saturated mono- or polycyclic aliphatic group which has between 3 and 24, between 3 and 16, between 3 and 14, between 3 and 12, between 3, 4, 5 or 6 carbon atoms and which is bound to the rest of the molecule by a single bond, including, for example and not restricted to, cyclopropyl, cyclobutyl, cyclopentyl, cyclohexyl, cycloheptyl, methyl cyclohexyl, dimethyl cyclohexyl, octahydroindene, decahydronaphthalene, dodecahydrophenalene and similar.

The term "cycloalkenyl" refers to a non-aromatic mono- or polycyclic aliphatic group which has between 5 and 24, between 5 and 16, between 5 and 14, between 5 and 12, 5 or 6 carbon atoms, with 1, 2 or 3 carbon-carbon double bonds, conjugated or unconjugated, and which is bound to the rest of the molecule by a single bond, including, for example and not restricted to, the cyclopent-1-en-1-yl group and similar.

The term "cycloalkynyl" refers to a non-aromatic mono- or polycyclic aliphatic group which has between 8 and 24, between 8 and 16, between 8 and 14, between 8 and 12, 8 or 9 carbon atoms, with 1, 2 or 3 carbon-carbon triple bonds, conjugated or unconjugated, and which is bound to the rest of the molecule by a single bond, including, for example and not restricted to, the cyclooct-2-in-1-yl group and similar.

The term "alkylamide" refers to an amide with at least one alkyl substituent.

The term "halosulfonate" refers to an ester group of sulfonic acid of formula Y-S(=O)₂-O- wherein Y is a halogen atom.

As used herein, the term "aprotic solvent" refers to a solvent which lacks an acidic proton.

The term "a" or "an" as used herein includes the singular and the plural, unless specifically stated otherwise. Therefore, the terms "a," "an" or "at least one" can be used interchangeably in this application.

For purposes of better understanding the present teachings and in no way limiting the scope of the teachings, unless otherwise indicated, all numbers expressing quantities, percentages or proportions, and other numerical values used in the specification and claims, are to be understood as being modified in all instances by the term "about." Accordingly, unless indicated to the contrary, the numerical parameters set forth in the following specification and attached claims are approximations that may vary depending upon the desired properties sought to be obtained. At the very least, each numerical parameter should at least be construed in light of the number of reported significant digits and by applying ordinary rounding techniques. In this regard, used of the term "about" herein specifically includes ±10% from the indicated values in the range. In addition, the endpoints of all ranges directed to the same component or property herein are inclusive of the endpoints, are independently combinable, and include all intermediate points and ranges. Similarly, the ranges and amounts for each element of the technology described herein can be used together with ranges or amounts for any of the other elements.

### Process for the preparation of biguanidine compounds

The present disclosure relates to a process for the preparation of a biguanidine compound of formula (I) or an acid addition salt thereof: 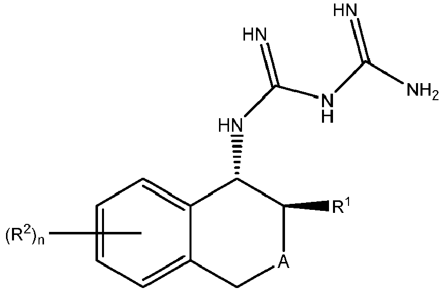
wherein R¹ and R² are each independently hydrogen or an optionally substituted C₁-C₄ alkyl group, wherein the optional substituents are selected from the group consisting of halogen, aliphatic, halo aliphatic, alicyclic, alkoxy, thioalkyl, cyano or nitro groups; A is -CH₂-, -O- or a direct bond; and n is 0, 1, 2 or 3;
which comprises the reaction of 1-cyanoguanidine with an amine of formula (II) or an acid addition salt thereof 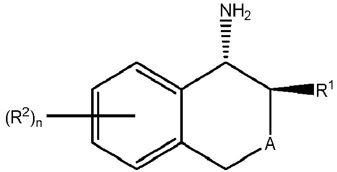
wherein R¹, R², A and n are as defined in formula (I), in a mixture which comprises a trimethylsilyl-containing compound, and an optionally alkyl substituted N-alkyl γ-, δ-, ε-lactam or an optionally alkyl substituted γ-, δ-, ε-lactone.

This process provides an alternative to the existing processes for the preparation of biguanidine compounds, and particularly, it provides a solution for some of the problems in the state of the art of low yield, considerable amounts of waste to be handled, high temperature of the reaction or operationally complicated isolation. The mixture of a trimethylsilyl-containing compound, and an optionally alkyl substituted N-alkyl γ-, δ-, ε-lactam or an optionally alkyl substituted γ-, δ-, ε-lactone allows to obtain a the biguanidide compound of formula (I) in a high yield. Additionally, we have found that the mixture of a trimethylsilyl-containing compound, and an optionally alkyl substituted N-alkyl γ-, δ-, ε-lactam or an optionally alkyl substituted γ-, δ-, ε-lactone allows to lower the reaction temperature from 140 ºC - 148 ºC, and to obtain a good yield for the synthesis of the biguanidide compound of formula (I).

Prior art document Obianom, O. N. et al., Molecular Pharmaceutics 2017, 14, 2726-2739*,* discloses the synthesis of 2,3-dihydro-1H-inden-2-yl-biguanidine HCl in acetonitrile with trimethylsilyl chloride at 120ºC with a yield of 9%.

We have found that the mixture of a trimethylsilyl-containing compound, and an optionally alkyl substituted N-alkyl γ-, δ-, ε-lactam or an optionally alkyl substituted γ-, δ-, ε-lactone, increases the yield of the biguanidine synthesis, and allows to lower the reaction temperature.

The R¹ and R² may be each independently C₁-C₄ alkyl group and n may be 1 in the process of the invention. The R¹ and R² may be each independently C₁-C₄ alkyl group, n may be 1, and A may be a direct bond in the process of the invention. The R¹ and R² may be methyl groups and n may be 1 in the process of the invention. The R¹ and R² may be methyl groups, n may be 1, and A may be a direct bond in the process of the invention. The compound of formula (I) in the process of the invention may be (1R,2S)-1-(bisguanidino)-2,6-dimethylindane or (1R,2S)-1-(bisguanidino)-2,6-dimethylindane monohydrochloride and the compound of formula (II) may be (1R,2S)-1-amino-2,6-dimethylindane or (1R,2S)-1-amino-2,6-dimethylindane monohydrochloride.

The acid addition salt of the biguanidine of formula (I) or the amine of formula (II) may be the salt of an acid such as for example, hydrogen chloride, hydrogen bromide, hydrogen iodide, phosphoric acid, sulfuric acid, nitric acid, carbonic acid, mono- or bifunctional carboxylic acids and hydroxycarboxylic acids, such as acetic acid, oxalic acid, maleic acid, succinic acid, fumaric acid, tartaric acid, citric acid, salicylic acid, sorbic acid or lactic acid, and also sulfonic acids, such as methanesulfonic acid, p-toluenesulfonic acid or 1,5-naphthalenedisulfonic acid. The acid addition salt of the amine of formula (II) may be produced by the reaction of the amine of formula (II) with the acid by any conventional method known by the person skilled in the art.

The temperature of the reaction in the process of the invention may range from 60 ºC to 100 ºC, from 65 ºC to 100 ºC, from 70 ºC to 100 ºC. When the temperature of the reaction in the process of the invention is lower than 60 ºC, the reaction is incomplete, or it starts to take a long time. When the temperature of the reaction is higher than 100 ºC, the yield of the biguanidide compound of formula (I) or an acid addition salt thereof starts to decrease due to the impurities that are produced. This reaction may be carried out at atmospheric pressure or a pressure higher than the atmospheric pressure.

The trimethylsilyl-containing compound, and the optionally alkyl substituted N-alkyl γ-, δ-, ε-lactam or the optionally alkyl substituted γ-, δ-, ε-lactone may be added dropwise or they may be added at once to the mixture of 1-cyanoguanidine with the amine of formula (II). The trimethylsilyl-containing compound, and the optionally alkyl substituted N-alkyl γ-, δ-, ε-lactam or the optionally alkyl substituted γ-, δ-, ε-lactone may be added together or separately.

The mole ratio between the trimethylsilyl-containing compound, and the optionally alkyl substituted N-alkyl γ-, δ-, ε-lactam or the optionally alkyl substituted γ-, δ-, ε-lactone may range from 1:1 to 1:20, or from 1:1.5 to 1:15. The mole ratio between the trimethylsilyl-containing compound, and the optionally alkyl substituted N-alkyl γ-, δ-, ε-lactam or the optionally alkyl substituted γ-, δ-, ε-lactone may range from 1:1 to 1:20, or from 1:1.5 to 1:15 and the temperature of the reaction may range from 60 ºC to 100 ºC. This reaction may be carried out at atmospheric pressure or a pressure higher than the atmospheric pressure.

The amount of the trimethylsilyl-containing compound may be at least 1 mole, at least 1.02 moles, at least 1.05 moles by mole of the amine of formula (II). The amount of the trimethylsilyl-containing compound may be at least 1 mole, at least 1.02 moles, at least 1.05 moles by mole of the amine of formula (II); and the mole ratio between the trimethylsilyl-containing compound, and the optionally alkyl substituted N-alkyl γ-, δ-, ε-lactam or the optionally alkyl substituted γ-, δ-, ε-lactone may range from 1:1 to 1:20, or from 1:1.5 to 1:15. The amount of the trimethylsilyl-containing compound may be at least 1 mole, at least 1.02 moles, at least 1.05 moles by mole of the amine of formula (II); the mole ratio between the trimethylsilyl-containing compound, and the optionally alkyl substituted N-alkyl γ-, δ-, ε-lactam or the optionally alkyl substituted γ-, δ-, ε-lactone may range from 1:1 to 1:20, or from 1:1.5 to 1:15; and the temperature of the reaction may range from 60 ºC to 100 ºC, from 65 ºC to 100 ºC, from 70 ºC to 100 ºC. The amount of the trimethylsilyl-containing compound may be at least 1.05 moles by mole of the amine of formula (II); the mole ratio between the trimethylsilyl-containing compound, and the optionally alkyl substituted N-alkyl γ-, δ-, ε-lactam or the optionally alkyl substituted γ-, δ-, ε-lactone may range from 1:1 to 1:20, or from 1:1.5 to 1:15; the mole ratio between the trimethylsilyl-containing compound, and the 1-cyanoguanidine may range from 0.6:1 to 1:0.6; and the temperature of the reaction may range from 60 ºC to 100 ºC. This reaction may be carried out at atmospheric pressure or a pressure higher than the atmospheric pressure.

The amount of the optionally alkyl substituted N-alkyl γ-, δ-, ε-lactam or the optionally alkyl substituted γ-, δ-, ε-lactone may be at least 1 mole, at least 1.5 moles, at least 2 moles by mole of the amine of formula (II). The amount of the optionally alkyl substituted N-alkyl γ-, δ-, ε-lactam or the optionally alkyl substituted γ-, δ-, ε-lactone may be at least 1 mole, at least 1.5 moles, at least 2 moles by mole of the amine of formula (II); and the mole ratio between the trimethylsilyl-containing compound, and the optionally alkyl substituted N-alkyl γ-, δ-, ε-lactam or the optionally alkyl substituted γ-, δ-, ε-lactone may range from 1:1 to 1:20, or from 1:1.5 to 1:15. The amount of the optionally alkyl substituted N-alkyl γ-, δ-, ε-lactam or the optionally alkyl substituted γ-, δ-, ε-lactone may be at least 1 mole, at least 1.5 moles, at least 2 moles by mole of the amine of formula (II); the mole ratio between the trimethylsilyl-containing compound, and the optionally alkyl substituted N-alkyl γ-, δ-, ε-lactam or the optionally alkyl substituted γ-, δ-, ε-lactone may range from 1:1 to 1:20, or from 1:1.5 to 1:15; and the temperature of the reaction may range from 60 ºC to 100 ºC, from 65 ºC to 100 ºC, from 70 ºC to 100 ºC. The amount of the optionally alkyl substituted N-alkyl γ-, δ-, ε-lactam or the optionally alkyl substituted γ-, δ-, ε-lactone may be at least 1 mole, at least 1.5 moles, at least 2 moles by mole of the amine of formula (II); the mole ratio between the trimethylsilyl-containing compound, and the optionally alkyl substituted N-alkyl γ-, δ-, ε-lactam or the optionally alkyl substituted γ-, δ-, ε-lactone may range from 1:1 to 1:20, or from 1:1.5 to 1:15; the mole ratio between the optionally alkyl substituted N-alkyl γ-, δ-, ε-lactam or the optionally alkyl substituted γ-, δ-, ε-lactone, and the 1-cyanoguanidine may range from 1:1 to 5:1, from 1.5:1 to 3:1; and the temperature of the reaction may range from 60 ºC to 100 ºC, from 65 ºC to 100 ºC, from 70 ºC to 100 ºC. The amount of the optionally alkyl substituted N-alkyl γ-, δ-, ε-lactam or the optionally alkyl substituted γ-, δ-, ε-lactone may be at least 1 mole, at least 1.5 moles, at least 2 moles by mole of the amine of formula (II); the mole ratio between the trimethylsilyl-containing compound, and the optionally alkyl substituted N-alkyl γ-, δ-, ε-lactam or the optionally alkyl substituted γ-, δ-, ε-lactone may range from 1:1 to 1:20, or from 1:1.5 to 1:15; the mole ratio between the optionally alkyl substituted N-alkyl γ-, δ-, ε-lactam or the optionally alkyl substituted γ-, δ-, ε-lactone, and the 1-cyanoguanidine may range from 1:1 to 5:1, from 1.5:1 to 3:1; the mole ratio between the trimethylsilyl-containing compound, and the 1-cyanoguanidine may range from 0.6:1 to 1:0.6, from 0.8:1 to 1:0.8; and the temperature of the reaction may range from 60 ºC to 100 ºC, from 65 ºC to 100 ºC, from 70 ºC to 100 ºC. The amount of the optionally alkyl substituted N-alkyl γ-, δ-, ε-lactam or the optionally alkyl substituted γ-, δ-, ε-lactone may be at least 1 mole by mole of the amine of formula (II); the mole ratio between the trimethylsilyl-containing compound, and the optionally alkyl substituted N-alkyl γ-, δ-, ε-lactam or the optionally alkyl substituted γ-, δ-, ε-lactone may range from 1:1 to 1:20, or from 1:1.5 to 1:15; the mole ratio between the optionally alkyl substituted N-alkyl γ-, δ-, ε-lactam or the optionally alkyl substituted γ-, δ-, ε-lactone, and the 1-cyanoguanidine may range from 1:1 to 5:1; the mole ratio between the trimethylsilyl-containing compound, and the 1-cyanoguanidine may range from 0.6:1 to 1:0.6; the amount of the trimethylsilyl-containing compound may be at least 1.05 moles by mole of the amine of formula (II); and the temperature of the reaction may range from 60 ºC to 100 ºC. This reaction may be carried out at atmospheric pressure or a pressure higher than the atmospheric pressure.

The trimethylsilyl-containing compound may be selected from the group consisting of trimethylsilyl halide, trimethylsilyl haloalkylsulfonate, trimetylsilyl halosulfonate, trimethylsilyl azide, trimethylsilyl cyanide, hexamethyldisilane, optionally halogen substituted alkyl trimethylsilane, trimethylsilylalkyne, tris(trimethylsilyl)silane, tris(trimethylsilyl)methane, tetrakis(trimethylsilyl)silane, optionally halogen substituted (trimethylsilyl)alkylamide, or secondary or tertiary (trimethylsilyl)amines. The trimethylsilyl-containing compound may be selected from the group consisting of trimethylsilyl chloride, trimethylsilyl iodide, trimethylsilyl bromide, trimetylsilyl trifluoromethanesulfonate, trimethylsilyl chlorosulfonate, trimethylsilyl azide, trimethylsilyl cyanide, hexamethyldisilane, tetrametylsilane, (trimethylsilyl)methyl chloride, 1-(trimethylsilyl)propyne, trimethylsilylacetylene, tris(trimethylsilyl)silane, tris(trimethylsilyl)methane, tetrakis(trimethylsilyl)silane, N,O-bis(trimethylsilyl)acetamide, N,O-bis(trimethylsilyl)trifluoroacetamide, N-methyl-N-(trimethylsilyl)trifluoroacetamide, N-(trimethylsilyl)diethylamine, bis(trimethylsilyl)amine or tris(trimethylsilyl)amine. The trimethylsilyl-containing compound may be selected from the group consisting of trimethylsilyl chloride, trimethylsilyl iodide, trimethylsilyl bromide, trimetylsilyl trifluoromethanesulfonate, N,O-bis(trimethylsilyl)acetamide, N,O-bis(trimethylsilyl)trifluoroacetamide, N-methyl-N-(trimethylsilyl)trifluoroacetamide, N-(trimethylsilyl)diethylamine, bis(trimethylsilyl)amine or tris(trimethylsilyl)amine. The trimethylsilyl-containing compound may be trimethylsilyl chloride.

The trimethylsilyl-containing compound may be selected from the group consisting of trimethylsilyl halide, trimethylsilyl haloalkylsulfonate, trimetylsilyl halosulfonate, trimethylsilyl azide, trimethylsilyl cyanide, hexamethyldisilane, optionally halogen substituted alkyl trimethylsilane, trimethylsilylalkyne, tris(trimethylsilyl)silane, tris(trimethylsilyl)methane, tetrakis(trimethylsilyl)silane, optionally halogen substituted (trimethylsilyl)alkylamide, or secondary or tertiary (trimethylsilyl)amines; and the temperature of the reaction may range from 60 ºC to 100 ºC, from 65 ºC to 100 ºC, from 70 ºC to 100 ºC. The trimethylsilyl-containing compound may be selected from the group consisting of trimethylsilyl chloride, trimethylsilyl iodide, trimethylsilyl bromide, trimetylsilyl trifluoromethanesulfonate, trimethylsilyl chlorosulfonate, trimethylsilyl azide, trimethylsilyl cyanide, hexamethyldisilane, tetrametylsilane, (trimethylsilyl)methyl chloride, 1-(trimethylsilyl)propyne, trimethylsilylacetylene, tris(trimethylsilyl)silane, tris(trimethylsilyl)methane, tetrakis(trimethylsilyl)silane, N,O-bis(trimethylsilyl)acetamide, N,O-bis(trimethylsilyl)trifluoroacetamide, N-methyl-N-(trimethylsilyl)trifluoroacetamide, N-(trimethylsilyl)diethylamine, bis(trimethylsilyl)amine or tris(trimethylsilyl)amine; the mole ratio between the trimethylsilyl-containing compound, and the optionally alkyl substituted N-alkyl γ-, δ-, ε-lactam or the optionally alkyl substituted γ-, δ-, ε-lactone may range from 1:1 to 1:20, or from 1:1.5 to 1:15; and the temperature of the reaction may range from 60 ºC to 100 ºC, from 65 ºC to 100 ºC, from 70 ºC to 100 ºC. The trimethylsilyl-containing compound may be selected from the group consisting of trimethylsilyl chloride, trimethylsilyl iodide, trimethylsilyl bromide, trimetylsilyl trifluoromethanesulfonate, N,O-bis(trimethylsilyl)acetamide, N,O-bis(trimethylsilyl)trifluoroacetamide, N-methyl-N-(trimethylsilyl)trifluoroacetamide, N-(trimethylsilyl)diethylamine, bis(trimethylsilyl)amine or tris(trimethylsilyl)amine; the mole ratio between the trimethylsilyl-containing compound, and the optionally alkyl substituted N-alkyl γ-, δ-, ε-lactam or the optionally alkyl substituted γ-, δ-, ε-lactone may range from 1:1 to 1:20, or from 1:1.5 to 1:15; the amount of the trimethylsilyl-containing compound may be at least 1 mole, at least 1.02 moles, at least 1.05 moles by mole of the amine of formula (II); and the temperature of the reaction may range from 60 ºC to 100 ºC, from 65 ºC to 100 ºC. The trimethylsilyl-containing compound may be trimethylsilyl chloride; the mole ratio between the trimethylsilyl-containing compound, and the optionally alkyl substituted N-alkyl γ-, δ-, ε-lactam or the optionally alkyl substituted γ-, δ-, ε-lactone may range from 1:1.5 to 1:15; the amount of the trimethylsilyl-containing compound may be at least 1.05 moles by mole of the amine of formula (II); the mole ratio between the trimethylsilyl-containing compound, and the 1-cyanoguanidine may range from 0.6:1 to 1:0.6; and the temperature of the reaction may range from 60 ºC to 100 ºC. This reaction may be carried out at atmospheric pressure or a pressure higher than the atmospheric pressure.

The optionally alkyl substituted N-alkyl γ-, δ-, ε-lactam or the optionally alkyl substituted γ-, δ-, ε-lactone may be selected from the group consisting of optionally alkyl substituted N-(C₁-C₁₈) alkyl pyrrolidones, optionally alkyl substituted N-(C₁-C₄) alkyl 2-piperidones, optionally alkyl substituted N-(C₁-C₄) alkyl caprolactams, optionally alkyl substituted γ-butyrolactones, optionally alkyl substituted δ-valerolactones, optionally alkyl substituted ε-caprolactones. The optionally alkyl substituted N-alkyl γ-, δ-, ε-lactam or the optionally alkyl substituted γ-, δ-, ε-lactone may be selected from the group consisting of N-methylpyrrolidone, N-ethyl-2-pyrrolidone, N-propyl-2-pyrrolidone, N-butyl-2-pyrrolidone, 1-tert-butylpyrrolidin-2-one, 1-pentylpyrrolidin-2-one, N-hexyl-2-pyrrolidinone, 1-octyl-2-pyrrolidone, N-decyl-2-pyrrolidone, 1-dodecyl-2-pyrrolidinone, 1-tetradecyl-2-pyrrolidinone, 5-methyl-1-tetradecyl-2-pyrrolidinone, 1-octadecyl-2-pyrrolidinone, 3-methyl-1-octadecyl-2-pyrrolidinone, N-methyl-2-piperidone, 1-ethyl-2-piperidinone, 1-propyl-2-piperidinone, 1-butyl-2-piperidinone, N-methyl caprolactam, N-ethyl caprolactam, γ-butyrolactone, γ-valerolactone, γ-caprolactone, γ-octalactone, γ-nonalactone, γ-decalactone, γ-undecalactone, δ-valerolactone, δ-octalactone, δ-decalactone, δ-undecalactone, ε-caprolactone, ε-decalactone or ε-dodecalactone. The optionally alkyl substituted N-alkyl γ-, δ-, ε-lactam or the optionally alkyl substituted γ-, δ-, ε-lactone may be selected from the group consisting of N-methylpyrrolidone, N-ethyl-2-pyrrolidone, N-propyl-2-pyrrolidone, N-butyl-2-pyrrolidone, N-methyl-2-piperidone, N-methyl caprolactam, N-ethyl caprolactam, γ-butyrolactone, δ-valerolactone or ε-caprolactone. The optionally alkyl substituted N-alkyl γ-, δ-, ε-lactam or the optionally alkyl substituted γ-, δ-, ε-lactone may be N-methylpyrrolidone.

The optionally alkyl substituted N-alkyl γ-, δ-, ε-lactam or the optionally alkyl substituted γ-, δ-, ε-lactone may be selected from the group consisting of optionally alkyl substituted N-(C₁-C₁₈) alkyl pyrrolidones, optionally alkyl substituted N-(C₁-C₄) alkyl 2-piperidones, optionally alkyl substituted N-(C₁-C₄) alkyl caprolactams, optionally alkyl substituted γ-butyrolactones, optionally alkyl substituted δ-valerolactones, optionally alkyl substituted ε-caprolactones; and the trimethylsilyl-containing compound may be selected from the group consisting of trimethylsilyl halide, trimethylsilyl haloalkylsulfonate, trimetylsilyl halosulfonate, trimethylsilyl azide, trimethylsilyl cyanide, hexamethyldisilane, optionally halogen substituted alkyl trimethylsilane, trimethylsilylalkyne, tris(trimethylsilyl)silane, tris(trimethylsilyl)methane, tetrakis(trimethylsilyl)silane, optionally halogen substituted (trimethylsilyl)alkylamide, or secondary or tertiary (trimethylsilyl)amines. The optionally alkyl substituted N-alkyl γ-, δ-, ε-lactam or the optionally alkyl substituted γ-, δ-, ε-lactone may be selected from the group consisting of N-methylpyrrolidone, N-ethyl-2-pyrrolidone, N-propyl-2-pyrrolidone, N-butyl-2-pyrrolidone, 1-tert-butylpyrrolidin-2-one, 1-pentylpyrrolidin-2-one, N-hexyl-2-pyrrolidinone, 1-octyl-2-pyrrolidone, N-decyl-2-pyrrolidone, 1-dodecyl-2-pyrrolidinone, 1-tetradecyl-2-pyrrolidinone, 5-methyl-1-tetradecyl-2-pyrrolidinone, 1-octadecyl-2-pyrrolidinone, 3-methyl-1-octadecyl-2-pyrrolidinone, N-methyl-2-piperidone, 1-ethyl-2-piperidinone, 1-propyl-2-piperidinone, 1-butyl-2-piperidinone, N-methyl caprolactam, N-ethyl caprolactam, γ-butyrolactone, γ-valerolactone, γ-caprolactone, γ-octalactone, γ-nonalactone, γ-decalactone, γ-undecalactone, δ-valerolactone, δ-octalactone, δ-decalactone, δ-undecalactone, ε-caprolactone, ε-decalactone or ε-dodecalactone; the trimethylsilyl-containing compound may be selected from the group consisting of trimethylsilyl chloride, trimethylsilyl iodide, trimethylsilyl bromide, trimetylsilyl trifluoromethanesulfonate, trimethylsilyl chlorosulfonate, trimethylsilyl azide, trimethylsilyl cyanide, hexamethyldisilane, tetrametylsilane, (trimethylsilyl)methyl chloride, 1-(trimethylsilyl)propyne, trimethylsilylacetylene, tris(trimethylsilyl)silane, tris(trimethylsilyl)methane, tetrakis(trimethylsilyl)silane, N,O-bis(trimethylsilyl)acetamide, N,O-bis(trimethylsilyl)trifluoroacetamide, N-methyl-N-(trimethylsilyl)trifluoroacetamide, N-(trimethylsilyl)diethylamine, bis(trimethylsilyl)amine or tris(trimethylsilyl)amine; and the temperature of the reaction may range from 60 ºC to 100 ºC, from 65 ºC to 100 ºC, from 70 ºC to 100 ºC. The optionally alkyl substituted N-alkyl γ-, δ-, ε-lactam or the optionally alkyl substituted γ-, δ-, ε-lactone may be selected from the group consisting of N-methylpyrrolidone, N-ethyl-2-pyrrolidone, N-propyl-2-pyrrolidone, N-butyl-2-pyrrolidone, N-methyl-2-piperidone, N-methyl caprolactam, N-ethyl caprolactam, γ-butyrolactone, δ-valerolactone or ε-caprolactone; the trimethylsilyl-containing compound may be selected from the group consisting of trimethylsilyl chloride, trimethylsilyl iodide, trimethylsilyl bromide, trimetylsilyl trifluoromethanesulfonate, N,O-bis(trimethylsilyl)acetamide, N,O-bis(trimethylsilyl)trifluoroacetamide, N-methyl-N-(trimethylsilyl)trifluoroacetamide, N-(trimethylsilyl)diethylamine, bis(trimethylsilyl)amine or tris(trimethylsilyl)amine; the mole ratio between the trimethylsilyl-containing compound, and the optionally alkyl substituted N-alkyl γ-, δ-, ε-lactam or the optionally alkyl substituted γ-, δ-, ε-lactone may range from 1:1 to 1:20, or from 1:1.5 to 1:15; and the temperature of the reaction may range from 60 ºC to 100 ºC, from 65 ºC to 100 ºC. The optionally alkyl substituted N-alkyl γ-, δ-, ε-lactam or the optionally alkyl substituted γ-, δ-, ε-lactone may be N-methylpyrrolidone; the trimethylsilyl-containing compound may be trimethylsilyl chloride; the mole ratio between the trimethylsilyl-containing compound, and the optionally alkyl substituted N-alkyl γ-, δ-, ε-lactam or the optionally alkyl substituted γ-, δ-, ε-lactone may range from 1:1 to 1:20, or from 1:1.5 to 1:15; the mole ratio between the optionally alkyl substituted N-alkyl γ-, δ-, ε-lactam or the optionally alkyl substituted γ-, δ-, ε-lactone, and the 1-cyanoguanidine may range from 1:1 to 5:1; and the temperature of the reaction may range from 60 ºC to 100 ºC. This reaction may be carried out at atmospheric pressure or a pressure higher than the atmospheric pressure.

In one embodiment, the process for the preparation of a biguanidine compound of formula (I) or an acid addition salt thereof may optionally contain an aprotic solvent. The aprotic solvent may be selected from the group consisting of optionally halogen substituted aromatic hydrocarbons, optionally halogen substituted aliphatic hydrocarbons, nitrogen heterocyclic compounds, optionally alkyl substituted cyclic ethers, aliphatic ethers, ethers of aromatic hydrocarbons, nitriles, ketones, esters or tertiary amides. The aprotic solvent may be selected from the group consisting of toluene, benzene, ethylbenzene, xylenes, cumene, cymenes, mesitylene, biphenyl, chlorobenzene, bromobenzene, o-dichlorobenzene, m-dichlorobenzene, p-dichlorobenzene, chloromethane, dichloromethane, trichloromethane, tetrachloromethane, dichloroethane, trichloroethane, trichloroethylene, tetrachloroethylene, pentane, hexane, heptane, cyclohexane, decalin, quinoline, isoquinoline, quinoxaline, phthalazine, quinazoline, cinnoline, pyridine, pyridazine, pyrimidine, pyrazine, triazines, 1,4-dioxane, tetrahydropyran, methyl tetrahydropyran, tetrahydrofuran, methyl tetrahydrofuran, dibutylether, tert-butyl methyl ether, anisole, acetonitrile, benzonitrile, acetone, butanone, ethyl acetate, n-butyl acetate, hexyl acetate, dimethylformamide or dimethylacetamide. The aprotic solvent may be selected from the group consisting of toluene, benzene, ethylbenzene, xylenes, cumene, cymenes, chlorobenzene, bromobenzene, o-dichlorobenzene, m-dichlorobenzene, p-dichlorobenzene, trichloromethane, tetrachloromethane, dichloroethane, trichloroethane, trichloroethylene, tetrachloroethylene, hexane, cyclohexane, quinoline, pyridine, 1,4-dioxane, tetrahydropyran, methyl tetrahydropyran, tetrahydrofuran, methyl tetrahydrofuran, anisole, acetonitrile, benzonitrile, acetone, ethyl acetate, dimethylformamide or dimethylacetamide. The aprotic solvent may be selected from the group consisting of toluene, chlorobenzene, 1,4-dioxane, tetrahydropyran, methyl tetrahydropyran, tetrahydrofuran, methyl tetrahydrofuran, anisole or acetonitrile.

The aprotic solvent may be selected from the group consisting of optionally halogen substituted aromatic hydrocarbons, optionally halogen substituted aliphatic hydrocarbons, nitrogen heterocyclic compounds, optionally alkyl substituted cyclic ethers, aliphatic ethers, ethers of aromatic hydrocarbons, nitriles, ketones, esters or tertiary amides; and the optionally alkyl substituted N-alkyl γ-, δ-, ε-lactam or the optionally alkyl substituted γ-, δ-, ε-lactone may be selected from the group consisting of optionally alkyl substituted N-(C₁-C₁₈) alkyl pyrrolidones, optionally alkyl substituted N-(C₁-C₄) alkyl 2-piperidones, optionally alkyl substituted N-(C₁-C₄) alkyl caprolactams, optionally alkyl substituted γ-butyrolactones, optionally alkyl substituted δ-valerolactones, optionally alkyl substituted ε-caprolactones. The aprotic solvent may be selected from the group consisting of toluene, benzene, ethylbenzene, xylenes, cumene, cymenes, mesitylene, biphenyl, chlorobenzene, bromobenzene, o-dichlorobenzene, m-dichlorobenzene, p-dichlorobenzene, chloromethane, dichloromethane, trichloromethane, tetrachloromethane, dichloroethane, trichloroethane, trichloroethylene, tetrachloroethylene, pentane, hexane, heptane, cyclohexane, decalin, quinoline, isoquinoline, quinoxaline, phthalazine, quinazoline, cinnoline, pyridine, pyridazine, pyrimidine, pyrazine, triazines, 1,4-dioxane, tetrahydropyran, methyl tetrahydropyran, tetrahydrofuran, methyl tetrahydrofuran, dibutylether, tert-butyl methyl ether, anisole, acetonitrile, benzonitrile, acetone, butanone, ethyl acetate, n-butyl acetate, hexyl acetate, dimethylformamide or dimethylacetamide; the optionally alkyl substituted N-alkyl γ-, δ-, ε-lactam or the optionally alkyl substituted γ-, δ-, ε-lactone may be selected from the group consisting of N-methylpyrrolidone, N-ethyl-2-pyrrolidone, N-propyl-2-pyrrolidone, N-butyl-2-pyrrolidone, 1-tert-butylpyrrolidin-2-one, 1-pentylpyrrolidin-2-one, N-hexyl-2-pyrrolidinone, 1-octyl-2-pyrrolidone, N-decyl-2-pyrrolidone, 1-dodecyl-2-pyrrolidinone, 1-tetradecyl-2-pyrrolidinone, 5-methyl-1-tetradecyl-2-pyrrolidinone, 1-octadecyl-2-pyrrolidinone, 3-methyl-1-octadecyl-2-pyrrolidinone, N-methyl-2-piperidone, 1-ethyl-2-piperidinone, 1-propyl-2-piperidinone, 1-butyl-2-piperidinone, N-methyl caprolactam, N-ethyl caprolactam, γ-butyrolactone, γ-valerolactone, γ-caprolactone, γ-octalactone, γ-nonalactone, γ-decalactone, γ-undecalactone, δ-valerolactone, δ-octalactone, δ-decalactone, δ-undecalactone, ε-caprolactone, ε-decalactone or ε-dodecalactone; and the trimethylsilyl-containing compound may be selected from the group consisting of trimethylsilyl chloride, trimethylsilyl iodide, trimethylsilyl bromide, trimetylsilyl trifluoromethanesulfonate, trimethylsilyl chlorosulfonate, trimethylsilyl azide, trimethylsilyl cyanide, hexamethyldisilane, tetrametylsilane, (trimethylsilyl)methyl chloride, 1-(trimethylsilyl)propyne, trimethylsilylacetylene, tris(trimethylsilyl)silane, tris(trimethylsilyl)methane, tetrakis(trimethylsilyl)silane, N,O-bis(trimethylsilyl)acetamide, N,O-bis(trimethylsilyl)trifluoroacetamide, N-methyl-N-(trimethylsilyl)trifluoroacetamide, N-(trimethylsilyl)diethylamine, bis(trimethylsilyl)amine or tris(trimethylsilyl)amine. The aprotic solvent may be selected from the group consisting of toluene, benzene, ethylbenzene, xylenes, cumene, cymenes, chlorobenzene, bromobenzene, o-dichlorobenzene, m-dichlorobenzene, p-dichlorobenzene, trichloromethane, tetrachloromethane, dichloroethane, trichloroethane, trichloroethylene, tetrachloroethylene, hexane, cyclohexane, quinoline, pyridine, 1,4-dioxane, tetrahydropyran, methyl tetrahydropyran, tetrahydrofuran, methyl tetrahydrofuran, anisole, acetonitrile, benzonitrile, acetone, ethyl acetate, dimethylformamide or dimethylacetamide; the optionally alkyl substituted N-alkyl γ-, δ-, ε-lactam or the optionally alkyl substituted γ-, δ-, ε-lactone may be selected from the group consisting of N-methylpyrrolidone, N-ethyl-2-pyrrolidone, N-propyl-2-pyrrolidone, N-butyl-2-pyrrolidone, N-methyl-2-piperidone, N-methyl caprolactam, N-ethyl caprolactam, γ-butyrolactone, δ-valerolactone or ε-caprolactone; the trimethylsilyl-containing compound may be selected from the group consisting of trimethylsilyl chloride, trimethylsilyl iodide, trimethylsilyl bromide, trimetylsilyl trifluoromethanesulfonate, N,O-bis(trimethylsilyl)acetamide, N,O-bis(trimethylsilyl)trifluoroacetamide, N-methyl-N-(trimethylsilyl)trifluoroacetamide, N-(trimethylsilyl)diethylamine, bis(trimethylsilyl)amine or tris(trimethylsilyl)amine; and the temperature of the reaction may range from 60 ºC to 100 ºC, from 65 ºC to 100 ºC. The aprotic solvent may be selected from the group consisting of toluene, chlorobenzene, 1,4-dioxane, tetrahydropyran, methyl tetrahydropyran, tetrahydrofuran, methyl tetrahydrofuran, anisole or acetonitrile; the optionally alkyl substituted N-alkyl γ-, δ-, ε-lactam or the optionally alkyl substituted γ-, δ-, ε-lactone may be N-methylpyrrolidone; the trimethylsilyl-containing compound may be trimethylsilyl chloride; the mole ratio between the trimethylsilyl-containing compound, and the optionally alkyl substituted N-alkyl γ-, δ-, ε-lactam or the optionally alkyl substituted γ-, δ-, ε-lactone may range from 1:1 to 1:20; and the temperature of the reaction may range from 60 ºC to 100 ºC. This reaction may be carried out at atmospheric pressure or a pressure higher than the atmospheric pressure.

The reaction in the process of the invention is carried out under a protective gas atmosphere.

### Process for the preparation of triazine compounds

The present invention also relates to a process for the preparation of a triazine compound of formula (IV): 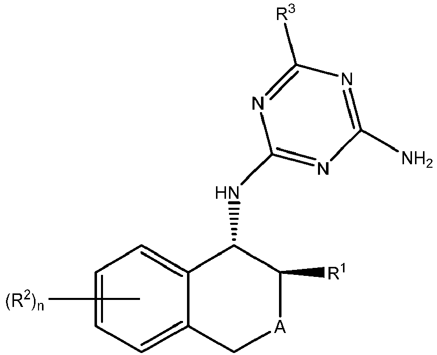 wherein R¹, R² and R³ are each independently hydrogen or an optionally substituted C₁-C₄ alkyl group, wherein the optional substituents are selected from the group consisting of halogen, aliphatic, halo aliphatic, alicyclic, alkoxy, thioalkyl, cyano or nitro groups; A is -CH₂-, -O- or a direct bond; and n is 0, 1, 2 or 3; which comprises
a) a first step of preparation of a biguanidine compound of formula (I) or an acid addition salt thereof: 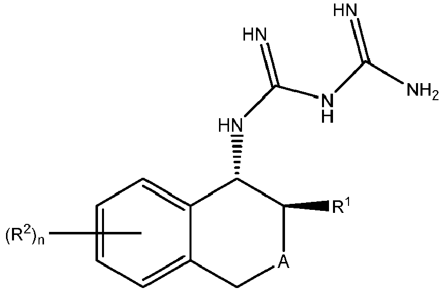
   wherein R¹, R², A and n are as defined in formula (IV);
   by reaction of 1-cyanoguanidine with an amine of formula (II) or an acid addition salt thereof 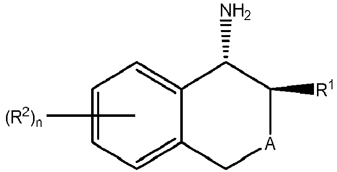
   wherein R¹, R², A and n are as defined in formula (IV), in a mixture which comprises a trimethylsilyl-containing compound, and an optionally alkyl substituted N-alkyl γ-, δ-, ε-lactam or an optionally alkyl substituted γ-, δ-, ε-lactone;
b) a base and a carboxylic acid derivative of formula (III) are added to the biguanidine compound of formula (I) or an acid addition salt thereof, obtained in step a)

   Z-R³ (III)

   wherein R³ is as defined in formula (IV), and Z-R³ is selected from the group consisting of carboxylic acid esters, carboxylic orthoesters, carboxylic acid chlorides, carboxamides, nitriles, or carboxylic anhydrides.

This process provides an alternative to the existing processes for the preparation of triazine compounds of formula (IV). The mixture of a trimethylsilyl-containing compound, and an optionally alkyl substituted N-alkyl γ-, δ-, ε-lactam or an optionally alkyl substituted γ-, δ-, ε-lactone allows to obtain a the biguanidide compound of formula (I) in a high yield in step a) of the process. Additionally, we have found that the mixture of solvents in step a) allows to lower the reaction temperature from 140 ºC - 148 ºC, and to carry out the preparation of triazine compounds of formula (IV) in a one-pot synthesis.

The R¹ and R² may be each independently C₁-C₄ alkyl group, n may be 1, R³ may be halogen substituted C₁-C₄ alkyl group, and Z-R³ may be a carboxylic ester. The R¹ and R² may be each independently C₁-C₄ alkyl group, n may be 1, A may be a direct bond, R³ may be halogen substituted C₁-C₄ alkyl group, and Z-R³ may be a carboxylic ester. The R¹ and R² may be methyl groups, n may be 1 and R³ may be (*R*)-2-fluoropropionate group. The R¹ and R² may be methyl groups, n may be 1, A may be a direct bond, R³ may be (*R*)-2-fluoropropionate group and Z-R³ may be methyl-(*R*)-2-fluoropropionate. The compound of formula (IV) in the process of the invention may be N-[(1*R*,2*S*)-2,6-dimethyl-2,3-dihydro-1H-inden-yl]-6-[(1*R*)-1-fluoroethyl]-1,3-5-triazine-2,4-diamine, the compound of formula (I) may be (1*R*,2*S*)-1-(bisguanidino)-2,6-dimethylindane or (1*R*,2*S*)-1-(bisguanidino)-2,6-dimethylindane monohydrochloride and the compound of formula (II) may be (1*R*,2*S*)-1-amino-2,6-dimethylindane or (1*R*,2*S*)-1-amino-2,6-dimethylindane monohydrochloride.

The mole ratio between the carboxylic acid derivative of formula (III) and the biguanidine compound of formula (I) may range from 1:1 to 5:1, from 1:1 to 3:1, or from 1:1 to 2:1.

The acid addition salt of the biguanidine of formula (I) or the amine of formula (II) in step a) may be the salt of an acid such as for example, hydrogen chloride, hydrogen bromide, hydrogen iodide, phosphoric acid, sulfuric acid, nitric acid, carbonic acid, mono- or bifunctional carboxylic acids and hydroxycarboxylic acids, such as acetic acid, oxalic acid, maleic acid, succinic acid, fumaric acid, tartaric acid, citric acid, salicylic acid, sorbic acid or lactic acid, and also sulfonic acids, such as methanesulfonic acid, p-toluenesulfonic acid or 1,5-naphthalenedisulfonic acid. The acid addition salt of the amine of formula (II) may be produced by the reaction of the amine of formula (II) with the acid by any conventional method known by the person skilled in the art.

The temperature of the reaction in step a) of the process of the invention may range from 60 ºC to 100 ºC, from 65 ºC to 100 ºC, from 70 ºC to 100 ºC. When the temperature of the reaction in the process of the invention is lower than 60 ºC, the reaction is incomplete, or it starts to take a long time. When the temperature of the reaction in step a) is higher than 100 ºC, the yield of the biguanidide compound of formula (I) or an acid addition salt thereof starts to decrease due to the impurities that are produced. The reaction of step a) may be carried out at atmospheric pressure or a pressure higher than the atmospheric pressure.

The trimethylsilyl-containing compound, and the optionally alkyl substituted N-alkyl γ-, δ-, ε-lactam or the optionally alkyl substituted γ-, δ-, ε-lactone may be added dropwise or they may be added at once to the mixture of 1-cyanoguanidine with the amine of formula (II) in step a) of the process of the invention. The trimethylsilyl-containing compound, and the optionally alkyl substituted N-alkyl γ-, δ-, ε-lactam or the optionally alkyl substituted γ-, δ-, ε-lactone may be added together or separately in step a) of the process of the invention.

The mole ratio between the trimethylsilyl-containing compound, and the optionally alkyl substituted N-alkyl γ-, δ-, ε-lactam or the optionally alkyl substituted γ-, δ-, ε-lactone may range from 1:1 to 1:20, or from 1:1.5 to 1:15 in step a) of the process of the invention. The mole ratio between the trimethylsilyl-containing compound, and the optionally alkyl substituted N-alkyl γ-, δ-, ε-lactam or the optionally alkyl substituted γ-, δ-, ε-lactone may range from 1:1 to 1:20, or from 1:1.5 to 1:15 and the temperature of the reaction may range from 60 ºC to 100 ºC in step a) of the process of the invention. The reaction of step a) may be carried out at atmospheric pressure or a pressure higher than the atmospheric pressure.

The amount of the trimethylsilyl-containing compound may be at least 1 mole, at least 1.02 moles, at least 1.05 moles by mole of the amine of formula (II) in step a) of the process of the invention. The amount of the trimethylsilyl-containing compound may be at least 1 mole, at least 1.02 moles, at least 1.05 moles by mole of the amine of formula (II); and the mole ratio between the trimethylsilyl-containing compound, and the optionally alkyl substituted N-alkyl γ-, δ-, ε-lactam or the optionally alkyl substituted γ-, δ-, ε-lactone may range from 1:1 to 1:20, or from 1:1.5 to 1:15 in step a) of the process of the invention. The amount of the trimethylsilyl-containing compound may be at least 1 mole, at least 1.02 moles, at least 1.05 moles by mole of the amine of formula (II); the mole ratio between the trimethylsilyl-containing compound, and the optionally alkyl substituted N-alkyl γ-, δ-, ε-lactam or the optionally alkyl substituted γ-, δ-, ε-lactone may range from 1:1 to 1:20, or from 1:1.5 to 1:15; and the temperature of the reaction may range from 60 ºC to 100 ºC, from 65 ºC to 100 ºC, from 70 ºC to 100 ºC in step a) of the process of the invention. The amount of the trimethylsilyl-containing compound may be at least 1.05 moles by mole of the amine of formula (II); the mole ratio between the trimethylsilyl-containing compound, and the optionally alkyl substituted N-alkyl γ-, δ-, ε-lactam or the optionally alkyl substituted γ-, δ-, ε-lactone may range from 1:1 to 1:20, or from 1:1.5 to 1:15; the mole ratio between the trimethylsilyl-containing compound, and the 1-cyanoguanidine may range from 0.6:1 to 1:0.6; and the temperature of the reaction may range from 60 ºC to 100 ºC in step a) of the process of the invention. The reaction of step a) may be carried out at atmospheric pressure or a pressure higher than the atmospheric pressure.

The amount of the optionally alkyl substituted N-alkyl γ-, δ-, ε-lactam or the optionally alkyl substituted γ-, δ-, ε-lactone may be at least 1 mole, at least 1.5 moles, at least 2 moles by mole of the amine of formula (II) in step a) of the process of the invention. The amount of the optionally alkyl substituted N-alkyl γ-, δ-, ε-lactam or the optionally alkyl substituted γ-, δ-, ε-lactone may be at least 1 mole, at least 1.5 moles, at least 2 moles by mole of the amine of formula (II); and the mole ratio between the trimethylsilyl-containing compound, and the optionally alkyl substituted N-alkyl γ-, δ-, ε-lactam or the optionally alkyl substituted γ-, δ-, ε-lactone may range from 1:1 to 1:20, or from 1:1.5 to 1:15 in step a) of the process of the invention. The amount of the optionally alkyl substituted N-alkyl γ-,δ-, ε-lactam or the optionally alkyl substituted γ-, δ-, ε-lactone may be at least 1 mole, at least 1.5 moles, at least 2 moles by mole of the amine of formula (II); the mole ratio between the trimethylsilyl-containing compound, and the optionally alkyl substituted N-alkyl γ-, δ-, ε-lactam or the optionally alkyl substituted γ-, δ-, ε-lactone may range from 1:1 to 1:20, or from 1:1.5 to 1:15; and the temperature of the reaction may range from 60 ºC to 100 ºC, from 65 ºC to 100 ºC, from 70 ºC to 100 ºC in step a) of the process of the invention. The amount of the optionally alkyl substituted N-alkyl γ-, δ-, ε-lactam or the optionally alkyl substituted γ-, δ-, ε-lactone may be at least 1 mole, at least 1.5 moles, at least 2 moles by mole of the amine of formula (II); the mole ratio between the trimethylsilyl-containing compound, and the optionally alkyl substituted N-alkyl γ-, δ-, ε-lactam or the optionally alkyl substituted γ-, δ-, ε-lactone may range from 1:1 to 1:20, or from 1:1.5 to 1:15; the mole ratio between the optionally alkyl substituted N-alkyl γ-, δ-, ε-lactam or the optionally alkyl substituted γ-, δ-, ε-lactone, and the 1-cyanoguanidine may range from 1:1 to 5:1, from 1.5:1 to 3:1; and the temperature of the reaction may range from 60 ºC to 100 ºC, from 65 ºC to 100 ºC, from 70 ºC to 100 ºC in step a) of the process of the invention. The amount of the optionally alkyl substituted N-alkyl γ-, δ-, ε-lactam or the optionally alkyl substituted γ-, δ-, ε-lactone may be at least 1 mole, at least 1.5 moles, at least 2 moles by mole of the amine of formula (II); the mole ratio between the trimethylsilyl-containing compound, and the optionally alkyl substituted N-alkyl γ-, δ-, ε-lactam or the optionally alkyl substituted γ-, δ-, ε-lactone may range from 1:1 to 1:20, or from 1:1.5 to 1:15; the mole ratio between the optionally alkyl substituted N-alkyl γ-, δ-, ε-lactam or the optionally alkyl substituted γ-, δ-, ε-lactone, and the 1-cyanoguanidine may range from 1:1 to 5:1, from 1.5:1 to 3:1; the mole ratio between the trimethylsilyl-containing compound, and the 1-cyanoguanidine may range from 0.6:1 to 1:0.6, from 0.8:1 to 1:0.8; and the temperature of the reaction may range from 60 ºC to 100 ºC, from 65 ºC to 100 ºC, from 70 ºC to 100 ºC in step a) of the process of the invention. The amount of the optionally alkyl substituted N-alkyl γ-, δ-, ε-lactam or the optionally alkyl substituted γ-, δ-, ε-lactone may be at least 1 mole by mole of the amine of formula (II); the mole ratio between the trimethylsilyl-containing compound, and the optionally alkyl substituted N-alkyl γ-, δ-, ε-lactam or the optionally alkyl substituted γ-, δ-, ε-lactone may range from 1:1 to 1:20, or from 1:1.5 to 1:15; the mole ratio between the optionally alkyl substituted N-alkyl γ-, δ-, ε-lactam or the optionally alkyl substituted γ-, δ-, ε-lactone, and the 1-cyanoguanidine may range from 1:1 to 5:1; the mole ratio between the trimethylsilyl-containing compound, and the 1-cyanoguanidine may range from 0.6:1 to 1:0.6; the amount of the trimethylsilyl-containing compound may be at least 1.05 moles by mole of the amine of formula (II); and the temperature of the reaction may range from 60 ºC to 100 ºC in step a) of the process of the invention. The reaction of step a) may be carried out at atmospheric pressure or a pressure higher than the atmospheric pressure.

The trimethylsilyl-containing compound may be selected from the group consisting of trimethylsilyl halide, trimethylsilyl haloalkylsulfonate, trimetylsilyl halosulfonate, trimethylsilyl azide, trimethylsilyl cyanide, hexamethyldisilane, optionally halogen substituted alkyl trimethylsilane, trimethylsilylalkyne, tris(trimethylsilyl)silane, tris(trimethylsilyl)methane, tetrakis(trimethylsilyl)silane, optionally halogen substituted (trimethylsilyl)alkylamide, or secondary or tertiary (trimethylsilyl)amines in step a) of the process of the invention. The trimethylsilyl-containing compound may be selected from the group consisting of trimethylsilyl chloride, trimethylsilyl iodide, trimethylsilyl bromide, trimetylsilyl trifluoromethanesulfonate, trimethylsilyl chlorosulfonate, trimethylsilyl azide, trimethylsilyl cyanide, hexamethyldisilane, tetrametylsilane, (trimethylsilyl)methyl chloride, 1-(trimethylsilyl)propyne, trimethylsilylacetylene, tris(trimethylsilyl)silane, tris(trimethylsilyl)methane, tetrakis(trimethylsilyl)silane, N,O-bis(trimethylsilyl)acetamide, N,O-bis(trimethylsilyl)trifluoroacetamide, N-methyl-N-(trimethylsilyl)trifluoroacetamide, N-(trimethylsilyl)diethylamine, bis(trimethylsilyl)amine or tris(trimethylsilyl)amine in step a) of the process of the invention. The trimethylsilyl-containing compound may be selected from the group consisting of trimethylsilyl chloride, trimethylsilyl iodide, trimethylsilyl bromide, trimetylsilyl trifluoromethanesulfonate, N,O-bis(trimethylsilyl)acetamide, N,O-bis(trimethylsilyl)trifluoroacetamide, N-methyl-N-(trimethylsilyl)trifluoroacetamide, N-(trimethylsilyl)diethylamine, bis(trimethylsilyl)amine or tris(trimethylsilyl)amine in step a) of the process of the invention. The trimethylsilyl-containing compound may be trimethylsilyl chloride in step a) of the process of the invention.

The trimethylsilyl-containing compound may be selected from the group consisting of trimethylsilyl halide, trimethylsilyl haloalkylsulfonate, trimetylsilyl halosulfonate, trimethylsilyl azide, trimethylsilyl cyanide, hexamethyldisilane, optionally halogen substituted alkyl trimethylsilane, trimethylsilylalkyne, tris(trimethylsilyl)silane, tris(trimethylsilyl)methane, tetrakis(trimethylsilyl)silane, optionally halogen substituted (trimethylsilyl)alkylamide, or secondary or tertiary (trimethylsilyl)amines; and the temperature of the reaction may range from 60 ºC to 100 ºC, from 65 ºC to 100 ºC, from 70 ºC to 100 ºC in step a) of the process of the invention. The trimethylsilyl-containing compound may be selected from the group consisting of trimethylsilyl chloride, trimethylsilyl iodide, trimethylsilyl bromide, trimetylsilyl trifluoromethanesulfonate, trimethylsilyl chlorosulfonate, trimethylsilyl azide, trimethylsilyl cyanide, hexamethyldisilane, tetrametylsilane, (trimethylsilyl)methyl chloride, 1-(trimethylsilyl)propyne, trimethylsilylacetylene, tris(trimethylsilyl)silane, tris(trimethylsilyl)methane, tetrakis(trimethylsilyl)silane, N,O-bis(trimethylsilyl)acetamide, N,O-bis(trimethylsilyl)trifluoroacetamide, N-methyl-N-(trimethylsilyl)trifluoroacetamide, N-(trimethylsilyl)diethylamine, bis(trimethylsilyl)amine or tris(trimethylsilyl)amine; the mole ratio between the trimethylsilyl-containing compound, and the optionally alkyl substituted N-alkyl γ-, δ-, ε-lactam or the optionally alkyl substituted γ-, δ-, ε-lactone may range from 1:1 to 1:20, or from 1:1.5 to 1:15; and the temperature of the reaction may range from 60 ºC to 100 ºC, from 65 ºC to 100 ºC, from 70 ºC to 100 ºC in step a) of the process of the invention. The trimethylsilyl-containing compound may be selected from the group consisting of trimethylsilyl chloride, trimethylsilyl iodide, trimethylsilyl bromide, trimetylsilyl trifluoromethanesulfonate, N,O-bis(trimethylsilyl)acetamide, N,O-bis(trimethylsilyl)trifluoroacetamide, N-methyl-N-(trimethylsilyl)trifluoroacetamide, N-(trimethylsilyl)diethylamine, bis(trimethylsilyl)amine or tris(trimethylsilyl)amine; the mole ratio between the trimethylsilyl-containing compound, and the optionally alkyl substituted N-alkyl γ-, δ-, ε-lactam or the optionally alkyl substituted γ-, δ-, ε-lactone may range from 1:1 to 1:20, or from 1:1.5 to 1:15; the amount of the trimethylsilyl-containing compound may be at least 1 mole, at least 1.02 moles, at least 1.05 moles by mole of the amine of formula (II); and the temperature of the reaction may range from 60 ºC to 100 ºC, from 65 ºC to 100 ºC in step a) of the process of the invention. The trimethylsilyl-containing compound may be trimethylsilyl chloride; the mole ratio between the trimethylsilyl-containing compound, and the optionally alkyl substituted N-alkyl γ-, δ-, ε-lactam or the optionally alkyl substituted γ-, δ-, ε-lactone may range from 1:1.5 to 1:15; the amount of the trimethylsilyl-containing compound may be at least 1.05 moles by mole of the amine of formula (II); the mole ratio between the trimethylsilyl-containing compound, and the 1-cyanoguanidine may range from 0.6:1 to 1:0.6; and the temperature of the reaction may range from 60 ºC to 100 ºC in step a) of the process of the invention. The reaction of step a) may be carried out at atmospheric pressure or a pressure higher than the atmospheric pressure.

The optionally alkyl substituted N-alkyl γ-, δ-, ε-lactam or the optionally alkyl substituted γ-, δ-, ε-lactone may be selected from the group consisting of optionally alkyl substituted N-(C₁-C₁₈) alkyl pyrrolidones, optionally alkyl substituted N-(C₁-C₄) alkyl 2-piperidones, optionally alkyl substituted N-(C₁-C₄) alkyl caprolactams, optionally alkyl substituted γ-butyrolactones, optionally alkyl substituted δ-valerolactones, optionally alkyl substituted ε-caprolactones, in step a) of the process of the invention. The optionally alkyl substituted N-alkyl γ-, δ-, ε-lactam or the optionally alkyl substituted γ-, δ-, ε-lactone may be selected from the group consisting of N-methylpyrrolidone, N-ethyl-2-pyrrolidone, N-propyl-2-pyrrolidone, N-butyl-2-pyrrolidone, 1-tert-butylpyrrolidin-2-one, 1-pentylpyrrolidin-2-one, N-hexyl-2-pyrrolidinone, 1-octyl-2-pyrrolidone, N-decyl-2-pyrrolidone, 1-dodecyl-2-pyrrolidinone, 1-tetradecyl-2-pyrrolidinone, 5-methyl-1-tetradecyl-2-pyrrolidinone, 1-octadecyl-2-pyrrolidinone, 3-methyl-1-octadecyl-2-pyrrolidinone, N-methyl-2-piperidone, 1-ethyl-2-piperidinone, 1-propyl-2-piperidinone, 1-butyl-2-piperidinone, N-methyl caprolactam, N-ethyl caprolactam, γ-butyrolactone, γ-valerolactone, γ-caprolactone, γ-octalactone, γ-nonalactone, γ-decalactone, γ-undecalactone, δ-valerolactone, δ-octalactone, δ-decalactone, δ-undecalactone, ε-caprolactone, ε-decalactone or ε-dodecalactone in step a) of the process of the invention. The optionally alkyl substituted N-alkyl γ-, δ-, ε-lactam or the optionally alkyl substituted γ-, δ-, ε-lactone may be selected from the group consisting of N-methylpyrrolidone, N-ethyl-2-pyrrolidone, N-propyl-2-pyrrolidone, N-butyl-2-pyrrolidone, N-methyl-2-piperidone, N-methyl caprolactam, N-ethyl caprolactam, γ-butyrolactone, δ-valerolactone or ε-caprolactone in step a) of the process of the invention. The optionally alkyl substituted N-alkyl γ-, δ-, ε-lactam or the optionally alkyl substituted γ-, δ-, ε-lactone may be N-methylpyrrolidone in step a) of the process of the invention.

The optionally alkyl substituted N-alkyl γ-, δ-, ε-lactam or the optionally alkyl substituted γ-, δ-, ε-lactone may be selected from the group consisting of optionally alkyl substituted N-(C₁-C₁₈) alkyl pyrrolidones, optionally alkyl substituted N-(C₁-C₄) alkyl 2-piperidones, optionally alkyl substituted N-(C₁-C₄) alkyl caprolactams, optionally alkyl substituted γ-butyrolactones, optionally alkyl substituted δ-valerolactones, optionally alkyl substituted ε-caprolactones; and the trimethylsilyl-containing compound may be selected from the group consisting of trimethylsilyl halide, trimethylsilyl haloalkylsulfonate, trimetylsilyl halosulfonate, trimethylsilyl azide, trimethylsilyl cyanide, hexamethyldisilane, optionally halogen substituted alkyl trimethylsilane, trimethylsilylalkyne, tris(trimethylsilyl)silane, tris(trimethylsilyl)methane, tetrakis(trimethylsilyl)silane, optionally halogen substituted (trimethylsilyl)alkylamide, or secondary or tertiary (trimethylsilyl)amines, in step a) of the process of the invention. The optionally alkyl substituted N-alkyl γ-, δ-, ε-lactam or the optionally alkyl substituted γ-, δ-, ε-lactone may be selected from the group consisting of N-methylpyrrolidone, N-ethyl-2-pyrrolidone, N-propyl-2-pyrrolidone, N-butyl-2-pyrrolidone, 1-tert-butylpyrrolidin-2-one, 1-pentylpyrrolidin-2-one, N-hexyl-2-pyrrolidinone, 1-octyl-2-pyrrolidone, N-decyl-2-pyrrolidone, 1-dodecyl-2-pyrrolidinone, 1-tetradecyl-2-pyrrolidinone, 5-methyl-1-tetradecyl-2-pyrrolidinone, 1-octadecyl-2-pyrrolidinone, 3-methyl-1-octadecyl-2-pyrrolidinone, N-methyl-2-piperidone, 1-ethyl-2-piperidinone, 1-propyl-2-piperidinone, 1-butyl-2-piperidinone, N-methyl caprolactam, N-ethyl caprolactam, γ-butyrolactone, γ-valerolactone, γ-caprolactone, γ-octalactone, γ-nonalactone, γ-decalactone, γ-undecalactone, δ-valerolactone, δ-octalactone, δ-decalactone, δ-undecalactone, ε-caprolactone, ε-decalactone or ε-dodecalactone; the trimethylsilyl-containing compound may be selected from the group consisting of trimethylsilyl chloride, trimethylsilyl iodide, trimethylsilyl bromide, trimetylsilyl trifluoromethanesulfonate, trimethylsilyl chlorosulfonate, trimethylsilyl azide, trimethylsilyl cyanide, hexamethyldisilane, tetrametylsilane, (trimethylsilyl)methyl chloride, 1-(trimethylsilyl)propyne, trimethylsilylacetylene, tris(trimethylsilyl)silane, tris(trimethylsilyl)methane, tetrakis(trimethylsilyl)silane, N,O-bis(trimethylsilyl)acetamide, N,O-bis(trimethylsilyl)trifluoroacetamide, N-methyl-N-(trimethylsilyl)trifluoroacetamide, N-(trimethylsilyl)diethylamine, bis(trimethylsilyl)amine or tris(trimethylsilyl)amine; and the temperature of the reaction may range from 60 ºC to 100 ºC, from 65 ºC to 100 ºC, from 70 ºC to 100 ºC in step a) of the process of the invention. The optionally alkyl substituted N-alkyl γ-, δ-, ε-lactam or the optionally alkyl substituted γ-, δ-, ε-lactone may be selected from the group consisting of N-methylpyrrolidone, N-ethyl-2-pyrrolidone, N-propyl-2-pyrrolidone, N-butyl-2-pyrrolidone, N-methyl-2-piperidone, N-methyl caprolactam, N-ethyl caprolactam, γ-butyrolactone, δ-valerolactone or ε-caprolactone; the trimethylsilyl-containing compound may be selected from the group consisting of trimethylsilyl chloride, trimethylsilyl iodide, trimethylsilyl bromide, trimetylsilyl trifluoromethanesulfonate, N,O-bis(trimethylsilyl)acetamide, N,O-bis(trimethylsilyl)trifluoroacetamide, N-methyl-N-(trimethylsilyl)trifluoroacetamide, N-(trimethylsilyl)diethylamine, bis(trimethylsilyl)amine or tris(trimethylsilyl)amine; the mole ratio between the trimethylsilyl-containing compound, and the optionally alkyl substituted N-alkyl γ-, δ-, ε-lactam or the optionally alkyl substituted γ-, δ-, ε-lactone may range from 1:1 to 1:20, or from 1:1.5 to 1:15; and the temperature of the reaction may range from 60 ºC to 100 ºC, from 65 ºC to 100 ºC in step a) of the process of the invention. The optionally alkyl substituted N-alkyl γ-, δ-, ε-lactam or the optionally alkyl substituted γ-, δ-, ε-lactone may be N-methylpyrrolidone; the trimethylsilyl-containing compound may be trimethylsilyl chloride; the mole ratio between the trimethylsilyl-containing compound, and the optionally alkyl substituted N-alkyl γ-, δ-, ε-lactam or the optionally alkyl substituted γ-, δ-, ε-lactone may range from 1:1 to 1:20, or from 1:1.5 to 1:15; the mole ratio between the optionally alkyl substituted N-alkyl γ-, δ-, ε-lactam or the optionally alkyl substituted γ-, δ-, ε-lactone, and the 1-cyanoguanidine may range from 1:1 to 5:1; and the temperature of the reaction may range from 60 ºC to 100 ºC in step a) of the process of the invention. The reaction of step a) may be carried out at atmospheric pressure or a pressure higher than the atmospheric pressure.

In one embodiment, the step a) for the preparation of a biguanidine compound of formula (I) or an acid addition salt thereof may optionally contain an aprotic solvent. The aprotic solvent may be selected from the group consisting of optionally halogen substituted aromatic hydrocarbons, optionally halogen substituted aliphatic hydrocarbons, nitrogen heterocyclic compounds, optionally alkyl substituted cyclic ethers, aliphatic ethers, ethers of aromatic hydrocarbons, nitriles, ketones, esters or tertiary amides, in step a) of the process of the invention. The aprotic solvent may be selected from the group consisting of toluene, benzene, ethylbenzene, xylenes, cumene, cymenes, mesitylene, biphenyl, chlorobenzene, bromobenzene, o-dichlorobenzene, m-dichlorobenzene, p-dichlorobenzene, chloromethane, dichloromethane, trichloromethane, tetrachloromethane, dichloroethane, trichloroethane, trichloroethylene, tetrachloroethylene, pentane, hexane, heptane, cyclohexane, decalin, quinoline, isoquinoline, quinoxaline, phthalazine, quinazoline, cinnoline, pyridine, pyridazine, pyrimidine, pyrazine, triazines, 1,4-dioxane, tetrahydropyran, methyl tetrahydropyran, tetrahydrofuran, methyl tetrahydrofuran, dibutylether, tert-butyl methyl ether, anisole, acetonitrile, benzonitrile, acetone, butanone, ethyl acetate, n-butyl acetate, hexyl acetate, dimethylformamide or dimethylacetamide in step a) of the process of the invention. The aprotic solvent may be selected from the group consisting of toluene, benzene, ethylbenzene, xylenes, cumene, cymenes, chlorobenzene, bromobenzene, o-dichlorobenzene, m-dichlorobenzene, p-dichlorobenzene, trichloromethane, tetrachloromethane, dichloroethane, trichloroethane, trichloroethylene, tetrachloroethylene, hexane, cyclohexane, quinoline, pyridine, 1,4-dioxane, tetrahydropyran, methyl tetrahydropyran, tetrahydrofuran, methyl tetrahydrofuran, anisole, acetonitrile, benzonitrile, acetone, ethyl acetate, dimethylformamide or dimethylacetamide in step a) of the process of the invention. The aprotic solvent may be selected from the group consisting of toluene, chlorobenzene, 1,4-dioxane, tetrahydropyran, methyl tetrahydropyran, tetrahydrofuran, methyl tetrahydrofuran, anisole or acetonitrile in step a) of the process of the invention.

The aprotic solvent may be selected from the group consisting of optionally halogen substituted aromatic hydrocarbons, optionally halogen substituted aliphatic hydrocarbons, nitrogen heterocyclic compounds, optionally alkyl substituted cyclic ethers, aliphatic ethers, ethers of aromatic hydrocarbons, nitriles, ketones, esters or tertiary amides; and the optionally alkyl substituted N-alkyl γ-, δ-, ε-lactam or the optionally alkyl substituted γ-, δ-, ε-lactone may be selected from the group consisting of optionally alkyl substituted N-(C₁-C₁₈) alkyl pyrrolidones, optionally alkyl substituted N-(C₁-C₄) alkyl 2-piperidones, optionally alkyl substituted N-(C₁-C₄) alkyl caprolactams, optionally alkyl substituted γ-butyrolactones, optionally alkyl substituted δ-valerolactones, optionally alkyl substituted ε-caprolactones, in step a) of the process of the invention. The aprotic solvent may be selected from the group consisting of toluene, benzene, ethylbenzene, xylenes, cumene, cymenes, mesitylene, biphenyl, chlorobenzene, bromobenzene, o-dichlorobenzene, m-dichlorobenzene, p-dichlorobenzene, chloromethane, dichloromethane, trichloromethane, tetrachloromethane, dichloroethane, trichloroethane, trichloroethylene, tetrachloroethylene, pentane, hexane, heptane, cyclohexane, decalin, quinoline, isoquinoline, quinoxaline, phthalazine, quinazoline, cinnoline, pyridine, pyridazine, pyrimidine, pyrazine, triazines, 1,4-dioxane, tetrahydropyran, methyl tetrahydropyran, tetrahydrofuran, methyl tetrahydrofuran, dibutylether, tert-butyl methyl ether, anisole, acetonitrile, benzonitrile, acetone, butanone, ethyl acetate, n-butyl acetate, hexyl acetate, dimethylformamide or dimethylacetamide; the optionally alkyl substituted N-alkyl γ-, δ-, ε-lactam or the optionally alkyl substituted γ-, δ-, ε-lactone may be selected from the group consisting of N-methylpyrrolidone, N-ethyl-2-pyrrolidone, N-propyl-2-pyrrolidone, N-butyl-2-pyrrolidone, 1-tert-butylpyrrolidin-2-one, 1-pentylpyrrolidin-2-one, N-hexyl-2-pyrrolidinone, 1-octyl-2-pyrrolidone, N-decyl-2-pyrrolidone, 1-dodecyl-2-pyrrolidinone, 1-tetradecyl-2-pyrrolidinone, 5-methyl-1-tetradecyl-2-pyrrolidinone, 1-octadecyl-2-pyrrolidinone, 3-methyl-1-octadecyl-2-pyrrolidinone, N-methyl-2-piperidone, 1-ethyl-2-piperidinone, 1-propyl-2-piperidinone, 1-butyl-2-piperidinone, N-methyl caprolactam, N-ethyl caprolactam, γ-butyrolactone, γ-valerolactone, γ-caprolactone, γ-octalactone, γ-nonalactone, γ-decalactone, γ-undecalactone, δ-valerolactone, δ-octalactone, δ-decalactone, δ-undecalactone, ε-caprolactone, ε-decalactone or ε-dodecalactone; and the trimethylsilyl-containing compound may be selected from the group consisting of trimethylsilyl chloride, trimethylsilyl iodide, trimethylsilyl bromide, trimetylsilyl trifluoromethanesulfonate, trimethylsilyl chlorosulfonate, trimethylsilyl azide, trimethylsilyl cyanide, hexamethyldisilane, tetrametylsilane, (trimethylsilyl)methyl chloride, 1-(trimethylsilyl)propyne, trimethylsilylacetylene, tris(trimethylsilyl)silane, tris(trimethylsilyl)methane, tetrakis(trimethylsilyl)silane, N,O-bis(trimethylsilyl)acetamide, N,O-bis(trimethylsilyl)trifluoroacetamide, N-methyl-N-(trimethylsilyl)trifluoroacetamide, N-(trimethylsilyl)diethylamine, bis(trimethylsilyl)amine or tris(trimethylsilyl)amine in step a) of the process of the invention. The aprotic solvent may be selected from the group consisting of toluene, benzene, ethylbenzene, xylenes, cumene, cymenes, chlorobenzene, bromobenzene, o-dichlorobenzene, m-dichlorobenzene, p-dichlorobenzene, trichloromethane, tetrachloromethane, dichloroethane, trichloroethane, trichloroethylene, tetrachloroethylene, hexane, cyclohexane, quinoline, pyridine, 1,4-dioxane, tetrahydropyran, methyl tetrahydropyran, tetrahydrofuran, methyl tetrahydrofuran, anisole, acetonitrile, benzonitrile, acetone, ethyl acetate, dimethylformamide or dimethylacetamide; the optionally alkyl substituted N-alkyl γ-, δ-, ε-lactam or the optionally alkyl substituted γ-, δ-, ε-lactone may be selected from the group consisting of N-methylpyrrolidone, N-ethyl-2-pyrrolidone, N-propyl-2-pyrrolidone, N-butyl-2-pyrrolidone, N-methyl-2-piperidone, N-methyl caprolactam, N-ethyl caprolactam, γ-butyrolactone, δ-valerolactone or ε-caprolactone; the trimethylsilyl-containing compound may be selected from the group consisting of trimethylsilyl chloride, trimethylsilyl iodide, trimethylsilyl bromide, trimetylsilyl trifluoromethanesulfonate, N,O-bis(trimethylsilyl)acetamide, N,O-bis(trimethylsilyl)trifluoroacetamide, N-methyl-N-(trimethylsilyl)trifluoroacetamide, N-(trimethylsilyl)diethylamine, bis(trimethylsilyl)amine or tris(trimethylsilyl)amine; and the temperature of the reaction may range from 60 ºC to 100 ºC, from 65 ºC to 100 ºC in step a) of the process of the invention. The aprotic solvent may be selected from the group consisting of toluene, chlorobenzene, 1,4-dioxane, tetrahydropyran, methyl tetrahydropyran, tetrahydrofuran, methyl tetrahydrofuran, anisole or acetonitrile; the optionally alkyl substituted N-alkyl γ-, δ-, ε-lactam or the optionally alkyl substituted γ-, δ-, ε-lactone may be N-methylpyrrolidone; the trimethylsilyl-containing compound may be trimethylsilyl chloride; the mole ratio between the trimethylsilyl-containing compound, and the optionally alkyl substituted N-alkyl γ-, δ-, ε-lactam or the optionally alkyl substituted γ-, δ-, ε-lactone may range from 1:1 to 1:20; and the temperature of the reaction may range from 60 ºC to 100 ºC in step a) of the process of the invention. The reaction of step a) may be carried out at atmospheric pressure or a pressure higher than the atmospheric pressure.

The process for the preparation of the triazine compound of formula (IV) may be carried out in one-pot synthesis without isolation of the biguanidine compound of formula (I) or an acid addition salt thereof, or it may be performed in 2 separate steps with isolation of the biguanidine compound of formula (I) or an acid addition salt thereof after step a).

In one embodiment, the biguanidine compound of formula (I) or an acid addition salt thereof may be isolated after step a) by any conventional method known by the person skilled in the art, including but not limited to filtration, washing the reaction product with a solvent or mixture of solvents in order to dissolve the impurities of the biguanidine compound of formula (I) or an acid addition salt thereof, or by crystallization of the biguanidine compound of formula (I) or an acid addition salt thereof.

In other embodiment, the biguanidine compound of formula (I) or an acid addition salt thereof is not isolated, then, the preparation of the triazine compound of formula (IV) is carried out in a one-pot synthesis, and the solvent from step a) is removed before the reaction of step b) of the process of the invention. The solvent may be removed by any conventional method known by the person skilled in the art, including but not limited to distillation, or distillation under vacuum.

The base in step b) of the process of the invention may be selected from the group consisting of an alkali metal, alkaline earth metal or ammonium hydroxide, hydride, alkoxide, carbonate, bicarbonate, phosphate, hydrogen- or dihydrogenphosphate, or a tertiary or aromatic amine or mixtures thereof. The base in step b) of the process of the invention may be selected from the group consisting of an alkali metal or alkaline earth metal hydroxide, alkoxide, carbonate, bicarbonate, or mixtures thereof. The base in step b) of the process of the invention may be selected from the group consisting of an alkali metal hydroxide, methoxide, ethoxide, propoxide, butoxide, carbonate or mixtures thereof.

The amount of the base in step b) of the process of the invention may range from 1 to 5 moles, from 1 to 4 moles, from 1 to 3 moles by mole of the biguanidine compound of formula (I).

The amount of the base in step b) of the process of the invention may range from 1 to 5 moles by mole of the biguanidine compound of formula (I) and the base may be selected from the group consisting of an alkali metal, alkaline earth metal or ammonium hydroxide, hydride, alkoxide, carbonate, bicarbonate, phosphate, hydrogen- or dihydrogenphosphate, or a tertiary or aromatic amine or mixtures thereof. The amount of the base in step b) of the process of the invention may range from 1 to 4 moles by mole of the biguanidine compound of formula (I) and the base may be selected from the group consisting of an alkali metal or alkaline earth metal hydroxide, alkoxide, carbonate, bicarbonate, or mixtures thereof. The amount of the base in step b) of the process of the invention may range from 1 to 3 moles by mole of the biguanidine compound of formula (I) and the base may be selected from the group consisting of an alkali metal hydroxide, methoxide, ethoxide, propoxide, butoxide, carbonate or mixtures thereof.

The solvent of the reaction in step b) may be any organic solvent. The solvent of the reaction in step b) may be selected from the group consisting of optionally halogen substituted aromatic hydrocarbons, optionally halogen substituted aliphatic hydrocarbons, nitrogen heterocyclic compounds, optionally alkyl substituted cyclic ethers, aliphatic ethers, ethers of aromatic hydrocarbons, nitriles, ketones, esters, amides, alcohols or glycols. The solvent of the reaction in step b) may be selected from the group consisting of toluene, benzene, ethylbenzene, xylenes, cumene, cymenes, mesitylene, biphenyl, chlorobenzene, bromobenzene, o-dichlorobenzene, m-dichlorobenzene, p-dichlorobenzene, chloromethane, dichloromethane, trichloromethane, tetrachloromethane, dichloroethane, trichloroethane, trichloroethylene, tetrachloroethylene, pentane, hexane, heptane, cyclohexane, decalin, quinoline, isoquinoline, quinoxaline, phthalazine, quinazoline, cinnoline, pyridine, pyridazine, pyrimidine, pyrazine, triazines, 1,4-dioxane, tetrahydropyran, methyl tetrahydropyran, tetrahydrofuran, methyl tetrahydrofuran, dibutylether, tert-butyl methyl ether, anisole, acetonitrile, benzonitrile, acetone, butanone, ethyl acetate, n-butyl acetate, hexyl acetate, dimethylformamide or dimethylacetamide, ethanol, methanol, butanol, tert-butanol, propanol, isopropyl alcohol, isoamyl alcohol, phenol, ethylene glycol, propylene glycol, diethylene glycol or dimethoxyethane. The solvent of the reaction in step b) may be selected from the group consisting of toluene, 1,4-dioxane, tetrahydropyran, methyl tetrahydropyran, tetrahydrofuran, methyl tetrahydrofuran, anisole, acetonitrile, dimethylformamide, dimethylacetamide, ethanol, methanol or butanol. The solvent of the reaction in step b) may be ethanol or methanol.

The temperature of step b) of the process of the invention may be from room temperature to the reflux temperature of the solvent in step b).

The steps a) and b) of the process of the invention are carried out under a protective gas atmosphere.

The following examples are included for illustrative purposes only and should not be construed as limitations on the invention claimed herein.

### Examples

### Example 1: Synthesis of N-[(1R,2S)-2,6-dimethyl-2,3-dihydro-1H-inden-yl]-6-[(1R)-1-fluoroethyl]-1,3-5-triazine-2,4-diamine with N-methylpyrrolidone and trimethylsilyl chloride at 90ºC.

To a three-necked round bottom flask equipped with a nitrogen inlet and a teflon coated magnetic stir bar, 112 ml of NMP (N-methylpyrrolidone) are charged under nitrogen atmosphere. TMSCI (trimethylsilyl chloride) (1.05 equiv, 105 mmol, 13.32 ml) is added and the reaction mixture is heated to 90 ºC. 2-cyanoguanidine (1.1 equiv, 110 mmol, 9.24 gr) and (1R,2S)-2,6-dimethyl-2,3-dihydro-1H-inden-1-amine (1 equiv, 100 mmol, 16.1 gr) in NMP (23ml) are added dropwise over 1.5 hours at that temperature during which a viscous off-white mixture is formed. Once addition of the components is completed, the reaction is mixed for 14 hours. Once termination of the 1st step is visible via HPLC, the reaction is cooled down to room temperature and methyl (*R*)-2-fluoropropanoate (1.7 equiv, 170 mmol, 18.02 gr) is added in one portion followed by a dropwise addition of NaOMe/MeOH solution (2.1 equiv, 210 mmol, 30%w/w, 37.82 gr). Once termination is visible, the mixture is transferred to a round bottom flask and concentrated under reduced pressure. The resulting solid wax is then recrystallized according to the following procedure: MeOH (5ml per gr of reactant amine of formula II) is added to the product and heated until full dissolution is visible. The mixture is stirred at room temperature and water (2.8ml per gr of reactant amine of formula II) is added dropwise over 0.5 hr followed by stirring for 2 additional hours (can also be left over night). During that time, formation of an off-white precipitate occurs. The resulting suspension is filtered, and the obtained off-white solid is washed twice with 100 ml of hot water. Finally, the material is dried in an oven at 70°C under vacuum. The chemical yield obtained is 70%.

### Example 2: Synthesis of N-[(1R,2S)-2,6-dimethyl-2,3-dihydro-1H-inden-yl]-6-[(1R)-1-fluoroethyl]-1,3-5-triazine-2,4-diamine with N-methylpyrrolidone and trimethylsilyl chloride at 100ºC.

To a three-necked round bottom flask equipped with a nitrogen inlet and a teflon coated magnetic stir bar, 112 ml of NMP (N-methylpyrrolidone) are charged under nitrogen atmosphere. TMSCI (1.05 equiv, 105 mmol, 13.32 ml) is added and the reaction mixture is heated to 100 ºC. A pre-prepared solution of 2-cyanoguanidine (1.1 equiv, 110 mmol, 9.24 gr) and (1*R*,2*S*)-2,6-dimethyl-2,3-dihydro-1H-inden-1-amine (1 equiv, 100 mmol, 16.1 gr) in NMP (23ml) is added dropwise over 1.5 hours at that temperature during which a viscous off-white mixture is formed. Once addition of the components is completed, the reaction is mixed for 12 hours.

Once termination of the 1st step is visible via HPLC, the reaction is cooled down to room temperature and methyl (R)-2-fluoropropanoate (1.7 equiv, 170 mmol, 18.02 gr) is added in one portion followed by a dropwise addition of NaOMe/MeOH solution (2.1 equiv, 210 mmol, 30%w/w, 37.82 gr). Once termination is visible, the mixture is transferred to a round bottom flask and concentrated under reduced pressure. The resulting solid wax is then recrystallized according to the following procedure: MeOH (5ml per gr of reactant amine of formula II) is added to the product and heated until full dissolution is visible. The mixture is stirred at room temperature and water (2.8ml per gr of reactant amine of formula II) is added dropwise over 0.5 hr followed by stirring for 2 additional hours (can also be left over night). During that time, formation of an off-white precipitate occurs. The resulting suspension is filtered, and the obtained off-white solid is washed twice with 100 ml of hot water. Finally, the material is dried in an oven at 70°C under vacuum. The chemical yield obtained is 56%.

### Example 3: Synthesis of N-[(1R,2S)-2,6-dimethyl-2,3-dihydro-1H-inden-yl]-6-[(1R)-1-fluoroethyl]-1,3-5-triazine-2,4-diamine with N-methylpyrrolidone, monochlorobenzene and trimethylsilyl chloride at 90ºC.

To a three-necked round bottom flask equipped with a nitrogen inlet and a teflon coated magnetic stir bar, 112 ml of monochlorobenzene are charged under nitrogen atmosphere. TMSCI (1.05 equiv, 105 mmol, 13.32 ml) is added and the reaction mixture is heated to 90 ºC. A pre-prepared solution of 2-cyanoguanidine (1.1 equiv, 110 mmol, 9.24 gr) and (1*R*,2*S*)-2,6-dimethyl-2,3-dihydro-1H-inden-1-amine (1 equiv, 100 mmol, 16.1 gr) in NMP (23ml) is added dropwise over 1.5 hours at that temperature during which a viscous off-white mixture is formed. Once addition of the components is completed, the reaction is mixed for 14 hours. Once termination of the 1st step is visible via HPLC, the reaction is cooled down to room temperature and methyl (*R*)-2-fluoropropanoate (1.7 equiv, 170 mmol, 18.02 gr) is added in one portion followed by a dropwise addition of NaOMe/MeOH solution (2.1 equiv, 210 mmol, 30%w/w, 37.82 gr). Once termination is visible, the mixture is transferred to a round bottom flask and concentrated under reduced pressure. The resulting solid wax is then recrystallized according to the following procedure: MeOH (5ml per gr of reactant amine of formula II) is added to the product and heated until full dissolution is visible. The mixture is stirred at room temperature and water (2.8ml per gr of reactant amine of formula II) is added dropwise over 0.5 hr followed by stirring for 2 additional hours (can also be left over night). During that time, formation of an off-white precipitate occurs. The resulting suspension is filtered, and the obtained off-white solid is washed twice with 100 ml of hot water. Finally, the material is dried in an oven at 70°C under vacuum. The chemical yield obtained is 85%.

### Example 4: Synthesis of N-[(1R,2S)-2,6-dimethyl-2,3-dihydro-1H-inden-yl]-6-[(1R)-1-fluoroethyl]-1,3-5-triazine-2,4-diamine with N-methylpyrrolidone, monochlorobenzene and trimethylsilyl chloride at 100ºC.

To a three-necked round bottom flask equipped with a nitrogen inlet and a teflon coated magnetic stir bar, 112 ml of monochlorobenzene are charged under nitrogen atmosphere. TMSCI (1.05 equiv, 105 mmol, 13.32 ml) is added and the reaction mixture is heated to 100 ºC. A pre-prepared solution of 2-cyanoguanidine (1.1 equiv, 110 mmol, 9.24 gr) and (1*R*,2*S*)-2,6-dimethyl-2,3-dihydro-1H-inden-1-amine (1 equiv, 100 mmol, 16.1 gr) in NMP (23ml) is added dropwise over 1.5 hours at that temperature during which a viscous off-white mixture is formed. Once addition of the components is completed, the reaction is mixed for 12-13 hours. Once termination of the 1st step is visible via HPLC, the reaction is cooled down to room temperature and methyl (*R*)-2-fluoropropanoate (1.7 equiv, 170 mmol, 18.02 gr) is added in one portion followed by a dropwise addition of NaOMe/MeOH solution (2.1 equiv, 210 mmol, 30%w/w, 37.82 gr). Once termination is visible, the mixture is transferred to a round bottom flask and concentrated under reduced pressure. The resulting solid wax is then recrystallized according to the following procedure: MeOH (5ml per gr of reactant amine of formula II) is added to the product and heated until full dissolution is visible. The mixture is stirred at room temperature and water (2.8ml per gr of reactant amine of formula II) is added dropwise over 0.5 hr followed by stirring for 2 additional hours (can also be left over night). During that time, formation of an off-white precipitate occurs. The resulting suspension is filtered, and the obtained off-white solid is washed twice with 100 ml of hot water. Finally, the material is dried in an oven at 70°C under vacuum. The chemical yield obtained is 85%.

### Example 5: Synthesis of N-[(1R,2S)-2,6-dimethyl-2,3-dihydro-1H-inden-yl]-6-[(1R)-1-fluoroethyl]-1,3-5-triazine-2,4-diamine with N-methylpyrrolidone, acetonitrile and trimethylsilyl chloride at 83ºC.

To a three-necked round bottom flask equipped with a nitrogen inlet and a teflon coated magnetic stir bar, 112 ml of acetonitrile are charged under nitrogen atmosphere. TMSCI (1.05 equiv, 105 mmol, 13.32 ml) is added and the reaction mixture is heated to 83 ºC. 2-cyanoguanidine (1.1 equiv, 110 mmol, 9.24 gr) and (1*R*,2*S*)-2,6-dimethyl-2,3-dihydro-1H-inden-1-amine (1 equiv, 100 mmol, 16.1 gr) in NMP (23ml) are added drop-wise over 1.5 hours at that temperature during which a viscous off-white mixture is formed. The mixture is mixed for 48 hours. Once termination of the 1st step is visible via HPLC, the reaction is cooled down to room temperature and methyl (*R*)-2-fluoropropanoate (1.7 equiv, 170 mmol, 18.02 gr) is added in one portion followed by a dropwise addition of NaOMe/MeOH solution (2.1 equiv, 210 mmol, 30%w/w, 37.82 gr). Once termination is visible, the mixture is transferred to a round bottom flask and concentrated under reduced pressure. The resulting solid wax is then recrystallized according to the following procedure: MeOH (5ml per gr of reactant amine of formula II) is added to the product and heated until full dissolution is visible. The mixture is stirred at room temperature and water (2.8ml per gr of reactant amine of formula II) is added dropwise over 0.5 hr followed by stirring for 2 additional hours (can also be left over night). During that time, formation of an off-white precipitate occurs. The resulting suspension is filtered, and the obtained off-white solid is washed twice with 100 ml of hot water. Finally, the material is dried in an oven at 70°C under vacuum. The chemical yield obtained is 85%.

### Comparative example 6: Synthesis of N-[(1R,2S)-2,6-dimethyl-2,3-dihydro-1H-inden-yl]-6-[(1R)-1-fluoroethyl]-1,3-5-triazine-2,4-diamine with acetonitrile and trimethylsilyl chloride at 83ºC.

To a three-necked round bottom flask equipped with a nitrogen inlet and a teflon coated magnetic stir bar, 112 ml of acetonitrile are charged under nitrogen atmosphere. TMSCI (1.05 equiv, 105 mmol, 13.32 ml) is added and the reaction mixture is heated to 83 ºC. 2-cyanoguanidine (1.1 equiv, 110 mmol, 9.24 gr) and (1*R*,2*S*)-2,6-dimethyl-2,3-dihydro-1H-inden-1-amine (1 equiv, 100 mmol, 16.1 gr) are added portion-wise over 1.5 hours at that temperature during which a coagulation occurs and the solution is not mixable. After 48 hours only a slight conversion is observed and thus the reaction is terminated.

### Example 7: Synthesis of N-[(1R,2S)-2,6-dimethyl-2,3-dihydro-1H-inden-yl]-6-[(1R)-1-fluoroethyl]-1,3-5-triazine-2,4-diamine with N-methylpyrrolidone, toluene and trimethylsilyl chloride at 90ºC.

To a three-necked round bottom flask equipped with a nitrogen inlet and a teflon coated magnetic stir bar, 112 ml of toluene are charged under nitrogen atmosphere. TMSCI (1.05 equiv, 105 mmol, 13.32 ml) is added and the reaction mixture is heated to 90ºC. A pre-prepared solution of 2-cyanoguanidine (1.1 equiv, 110 mmol, 9.24 gr) and (1*R*,2*S*)-2,6-dimethyl-2,3-dihydro-1H-inden-1-amine (1 equiv, 100 mmol, 16.1 gr) in NMP (23ml) is added dropwise over 1.5 hours at that temperature during which a viscous off-white mixture is formed. Once addition of the components is completed, the reaction is mixed for 14 hours. Once termination of the 1st step is visible via HPLC, the reaction is cooled down to room temperature and methyl (*R*)-2-fluoropropanoate (1.7 equiv, 170 mmol, 18.02 gr) is added in one portion followed by a dropwise addition of NaOMe/MeOH solution (2.1 equiv, 210 mmol, 30%w/w, 37.82 gr). Once termination is visible, the mixture is transferred to a round bottom flask and concentrated under reduced pressure. The resulting solid wax is then recrystallized according to the following procedure: MeOH (5ml per gr of reactant amine of formula II) is added to the product and heated until full dissolution is visible. The mixture is stirred at room temperature and water (2.8ml per gr of reactant amine of formula II) is added dropwise over 0.5 hr followed by stirring for 2 additional hours (can also be left over night). During that time, formation of an off-white precipitate occurs. The resulting suspension is filtered, and the obtained off-white solid is washed twice with 100 ml of hot water. Finally, the material is dried in an oven at 70°C under vacuum. The chemical yield obtained is 71%.

### Example 8: Synthesis of N-[(1R,2S)-2,6-dimethyl-2,3-dihydro-1H-inden-yl]-6-[(1R)-1-fluoroethyl]-1,3-5-triazine-2,4-diamine with N-methylpyrrolidone, toluene and trimethylsilyl chloride at 100ºC.

To a three-necked round bottom flask equipped with a nitrogen inlet and a teflon coated magnetic stir bar, 112 ml of toluene are charged under nitrogen atmosphere. TMSCI (1.05 equiv, 105 mmol, 13.32 ml) is added and the reaction mixture is heated to 100 ºC. A pre-prepared solution of 2-cyanoguanidine (1.1 equiv, 110 mmol, 9.24 gr) and (1*R*,2*S*)-2,6-dimethyl-2,3-dihydro-1H-inden-1-amine (1 equiv, 100 mmol, 16.1 gr) in NMP (23ml) is added dropwise over 1.5 hours at that temperature during which a viscous off-white mixture is formed. Once addition of the components is completed, the reaction is mixed for 48 hours. Once termination of the 1st step is visible via HPLC, the reaction is cooled down to room temperature and methyl (*R*)-2-fluoropropanoate (1.7 equiv, 170 mmol, 18.02 gr) is added in one portion followed by a dropwise addition of NaOMe/MeOH solution (2.1 equiv, 210 mmol, 30%w/w, 37.82 gr). Once termination is visible, the mixture is transferred to a round bottom flask and concentrated under reduced pressure. The resulting solid wax is then recrystallized according to the following procedure: MeOH (5ml per gr of reactant amine of formula II) is added to the product and heated until full dissolution is visible. The mixture is stirred at room temperature and water (2.8ml per gr of reactant amine of formula II) is added dropwise over 0.5 hr followed by stirring for 2 additional hours (can also be left over night). During that time, formation of an off-white precipitate occurs. The resulting suspension is filtered, and the obtained off-white solid is washed twice with 100 ml of hot water. Finally, the material is dried in an oven at 70°C under vacuum. The chemical yield obtained is 50%.

### Comparative example 9: Synthesis of N-[(1R,2S)-2,6-dimethyl-2,3-dihydro-1H-inden-yl]-6-[(1R)-1-fluoroethyl]-1,3-5-triazine-2,4-diamine with toluene and trimethylsilyl chloride at 90ºC.

To a three-necked round bottom flask equipped with a nitrogen inlet and a teflon coated magnetic stir bar, 112 ml of toluene are charged under nitrogen atmosphere. TMSCI (1.05 equiv, 105 mmol, 13.32 ml) is added and the reaction mixture is heated to 90 ºC. 2-cyanoguanidine (1.1 equiv, 110 mmol, 9.24 gr) and (1*R*,2*S*)-2,6-dimethyl-2,3-dihydro-1H-inden-1-amine (1 equiv, 100 mmol, 16.1 gr) are added portion wise over 1.5 hours at that temperature during which a viscous off-white mixture is formed. The mixture is mixed for 48 hours but no termination of the 1st step is visible. The reaction is therefore discontinued.

### Example 10: Synthesis of N-[(1R,2S)-2,6-dimethyl-2,3-dihydro-1H-inden-yl]-6-[(1R)-1-fluoroethyl]-1,3-5-triazine-2,4-diamine with N-methylpyrrolidone, dioxan and trimethylsilyl chloride at 90ºC.

To a three-necked round bottom flask equipped with a nitrogen inlet and a teflon coated magnetic stir bar, 112 ml of dioxan are charged under nitrogen atmosphere. TMSCI (1.05 equiv, 105 mmol, 13.32 ml) is added and the reaction mixture is heated to 90 ºC. A pre-prepared solution of 2-cyanoguanidine (1.1 equiv, 110 mmol, 9.24 gr) and (1*R*,2*S*)-2,6-dimethyl-2,3-dihydro-1H-inden-1-amine (1 equiv, 100 mmol, 16.1 gr) in NMP (23ml) is added dropwise over 1.5 hours at that temperature during which a viscous off-white mixture is formed. Once addition of the components is completed, the reaction is mixed for 14 hours. Once termination of the 1st step is visible via HPLC, the reaction is cooled down to room temperature and methyl (*R*)-2-fluoropropanoate (1.7 equiv, 170 mmol, 18.02 gr) is added in one portion followed by a dropwise addition of NaOMe/MeOH solution (2.1 equiv, 210 mmol, 30%w/w, 37.82 gr). Once termination is visible, the mixture is transferred to a round bottom flask and concentrated under reduced pressure. The resulting solid wax is then recrystallized according to the following procedure: MeOH (5ml per gr of reactant amine of formula II) is added to the product and heated until full dissolution is visible. The mixture is stirred at room temperature and water (2.8ml per gr of reactant amine of formula II) is added dropwise over 0.5 hr followed by stirring for 2 additional hours (can also be left over night). During that time, formation of an off-white precipitate occurs. The resulting suspension is filtered, and the obtained off-white solid is washed twice with 100 ml of hot water. Finally, the material is dried in an oven at 70°C under vacuum. The chemical yield obtained is 79%.

### Example 11: Synthesis of N-[(1R,2S)-2,6-dimethyl-2,3-dihydro-1H-inden-yl]-6-[(1R)-1-fluoroethyl]-1,3-5-triazine-2,4-diamine with N-methylpyrrolidone, anisole and trimethylsilyl chloride at 90ºC.

To a three-necked round bottom flask equipped with a nitrogen inlet and a teflon coated magnetic stir bar, 112 ml of anisole are charged under nitrogen atmosphere. TMSCI (1.05 equiv, 105 mmol, 13.32 ml) is added and the reaction mixture is heated to 90 ºC. A pre-prepared solution of 2-cyanoguanidine (1.1 equiv, 110 mmol, 9.24 gr) and (1*R*,2*S*)-2,6-dimethyl-2,3-dihydro-1H-inden-1-amine (1 equiv, 100 mmol, 16.1 gr) in NMP (23ml) is added dropwise over 1.5 hours at that temperature during which a viscous off-white mixture is formed. Once addition of the components is completed, the reaction is mixed for 48 hours. Once termination of the 1st step is visible via HPLC, the reaction is cooled down to room temperature and methyl (*R*)-2-fluoropropanoate (1.7 equiv, 170 mmol, 18.02 gr) is added in one portion followed by a dropwise addition of NaOMe/MeOH solution (2.1 equiv, 210 mmol, 30%w/w, 37.82 gr). Once termination is visible, the mixture is transferred to a round bottom flask and concentrated under reduced pressure. The resulting solid wax is then recrystallized according to the following procedure: MeOH (5ml per gr of reactant amine of formula II) is added to the product and heated until full dissolution is visible. The mixture is stirred at room temperature and water (2.8ml per gr of reactant amine of formula II) is added dropwise over 0.5 hr followed by stirring for 2 additional hours (can also be left over night). During that time, formation of an off-white precipitate occurs. The resulting suspension is filtered, and the obtained off-white solid is washed twice with 100 ml of hot water. Finally, the material is dried in an oven at 70°C under vacuum. The chemical yield obtained is 84%.

### Example 12: Synthesis of N-[(1R,2S)-2,6-dimethyl-2,3-dihydro-1H-inden-yl]-6-[(1R)-1-fluoroethyl]-1,3-5-triazine-2,4-diamine with N-methylpyrrolidone, methyl tetrahydropyran and trimethylsilyl chloride at 90ºC.

To a three-necked round bottom flask equipped with a nitrogen inlet and a teflon coated magnetic stir bar, 112 ml of Me-THP (methyl tetrahydropyran) are charged under nitrogen atmosphere. TMSCI (1.05 equiv, 105 mmol, 13.32 ml) is added and the reaction mixture is heated to 90 ºC. A pre-prepared solution of 2-cyanoguanidine (1.1 equiv, 110 mmol, 9.24 gr) and (1*R*,2*S*)-2,6-dimethyl-2,3-dihydro-1H-inden-1-amine (1 equiv, 100 mmol, 16.1 gr) in NMP (23ml) is added dropwise over 1.5 hours at that temperature during which a viscous off-white mixture is formed. Once addition of the components is completed, the reaction is mixed for 14 hours. Once termination of the 1st step is visible via HPLC, the reaction is cooled down to room temperature and methyl (*R*)-2-fluoropropanoate (1.7 equiv, 170 mmol, 18.02 gr) is added in one portion followed by a dropwise addition of NaOMe/MeOH solution (2.1 equiv, 210 mmol, 30%w/w, 37.82 gr). Once termination is visible, the mixture is transferred to a round bottom flask and concentrated under reduced pressure. The resulting solid wax is then recrystallized according to the following procedure: MeOH (5ml per gr of reactant amine of formula II) is added to the product and heated until full dissolution is visible. The mixture is stirred at room temperature and water (2.8ml per gr of reactant amine of formula II) is added dropwise over 0.5 hr followed by stirring for 2 additional hours (can also be left over night). During that time, formation of an off-white precipitate occurs. The resulting suspension is filtered, and the obtained off-white solid is washed twice with 100 ml of hot water. Finally, the material is dried in an oven at 70°C under vacuum. The chemical yield obtained is 84%.

## Claims

1. A process for the preparation of a biguanidine compound of formula (I) or an acid addition salt thereof: 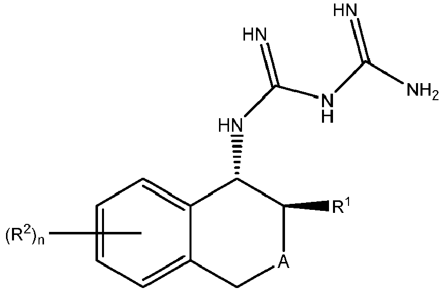
wherein R¹ and R² are each independently hydrogen or an optionally substituted C₁-C₄ alkyl group, wherein the optional substituents are selected from the group consisting of halogen, aliphatic, halo aliphatic, alicyclic, alkoxy, thioalkyl, cyano or nitro groups; A is -CH₂-, -O- or a direct bond; and n is 0, 1, 2 or 3;
which comprises the reaction of 1-cyanoguanidine with an amine of formula (II) or an acid addition salt thereof 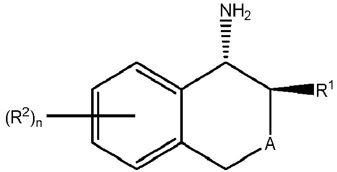
wherein R¹, R², A and n are as defined in formula (I), in a mixture which comprises a trimethylsilyl-containing compound, and an optionally alkyl substituted N-alkyl γ-, δ-, ε-lactam or an optionally alkyl substituted γ-, δ-, ε-lactone.

2. The process according to claim 1, wherein R¹ and R² are each independently C₁-C₄ alkyl group and n is 1.

3. The process according to any of previous claims, wherein the compound of formula (I) is (1*R*,2*S*)-1-(bisguanidino)-2,6-dimethylindane or (1*R*,2*S*)-1-(bisguanidino)-2,6-dimethylindane monohydrochloride and the compound of formula (II) is (1*R*,2*S*)-1-amino-2,6-dimethylindane or (1*R*,2*S*)-1-amino-2,6-dimethylindane monohydrochloride.

4. The process according to any of previous claims, wherein the temperature of the reaction ranges from 60 ºC to 100 ºC.

5. The process according to any of previous claims, wherein the mole ratio between the trimethylsilyl-containing compound, and the optionally alkyl substituted N-alkyl γ-, δ-, ε-lactam or the optionally alkyl substituted γ-, δ-, ε-lactone ranges from 1:1 to 1:20.

6. The process according to any of previous claims, wherein the trimethylsilyl-containing compound is selected from the group consisting of trimethylsilyl halide, trimethylsilyl haloalkylsulfonate, trimetylsilyl halosulfonate, trimethylsilyl azide, trimethylsilyl cyanide, hexamethyldisilane, optionally halogen substituted alkyl trimethylsilane, trimethylsilylalkyne, tris(trimethylsilyl)silane, tris(trimethylsilyl)methane, tetrakis(trimethylsilyl)silane, optionally halogen substituted (trimethylsilyl)alkylamide, or secondary or tertiary (trimethylsilyl)amines.

7. The process according to any of previous claims, wherein the optionally alkyl substituted N-alkyl γ-, δ-, ε-lactam or the optionally alkyl substituted γ-, δ-, ε-lactone is selected from the group consisting of optionally alkyl substituted N-(C₁-C₁₈) alkyl pyrrolidones, optionally alkyl substituted N-(C₁-C₄) alkyl 2-piperidones, optionally alkyl substituted N-(C₁-C₄) alkyl caprolactams, optionally alkyl substituted γ-butyrolactones, optionally alkyl substituted δ-valerolactones, optionally alkyl substituted ε-caprolactones.

8. The process according to any of previous claims, wherein further contains an aprotic solvent.

9. A process for the preparation of a triazine compound of formula (IV): 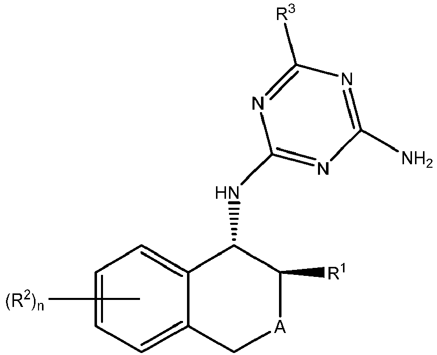 wherein R¹, R² and R³ are each independently hydrogen or an optionally substituted C₁-C₄ alkyl group, wherein the optional substituents are selected from the group consisting of halogen, aliphatic, halo aliphatic, alicyclic, alkoxy, thioalkyl, cyano or nitro groups; A is -CH₂-, -O- or a direct bond; and n is 0, 1, 2 or 3; which comprises
a) a first step of preparation of a biguanidine compound of formula (I) or an acid addition salt thereof according to any of previous claims;
b) a base and a carboxylic acid derivative of formula (III) are added to the biguanidine compound of formula (I) or an acid addition salt thereof, obtained in step a)
Z-R³ (III)
wherein R³ is as defined in formula (IV), and Z-R³ is selected from the group consisting of carboxylic acid esters, carboxylic orthoesters, carboxylic acid chlorides, carboxamides, nitriles, or carboxylic anhydrides.

10. The process according to claim 9, wherein R¹ and R² are each independently C₁-C₄ alkyl group, n is 1, R³ is halogen substituted C₁-C₄ alkyl group, and Z-R³ is a carboxylic ester.

11. The process according to any of claims 9 to 10, wherein the compound of formula (IV) is N-[(1*R*,2*S*)-2,6-dimethyl-2,3-dihydro-1H-inden-yl]-6-[(1*R*)-1-fluoroethyl]-1,3-5-triazine-2,4-diamine, the compound of formula (I) is (1*R*,2*S*)-1-(bisguanidino)-2,6-dimethylindane or (1*R*,2*S*)-1-(bisguanidino)-2,6-dimethylindane monohydrochloride and the compound of formula (II) is (1*R*,2*S*)-1-amino-2,6-dimethylindane or (1*R*,2*S*)-1-amino-2,6-dimethylindane monohydrochloride.

12. The process according to any of claims 9 to 11, wherein the temperature of the reaction in step a) ranges from 60 ºC to 100 ºC.

13. The process according to any of claims 9 to 12, wherein the trimethylsilyl-containing compound in step a) is selected from the group consisting of trimethylsilyl halide, trimethylsilyl haloalkylsulfonate, trimetylsilyl halosulfonate, trimethylsilyl azide, trimethylsilyl cyanide, hexamethyldisilane, optionally halogen substituted alkyl trimethylsilane, trimethylsilylalkyne, tris(trimethylsilyl)silane, tris(trimethylsilyl)methane, tetrakis(trimethylsilyl)silane, optionally halogen substituted (trimethylsilyl)alkylamide, or secondary or tertiary (trimethylsilyl)amines.

14. The process according to any of claims 9 to 13, wherein the optionally alkyl substituted N-alkyl γ-, δ-, ε-lactam or the optionally alkyl substituted γ-, δ-, ε-lactone in step a) is selected from the group consisting of optionally alkyl substituted N-(C₁-C₁₈) alkyl pyrrolidones, optionally alkyl substituted N-(C₁-C₄) alkyl 2-piperidones, optionally alkyl substituted N-(C₁-C₄) alkyl caprolactams, optionally alkyl substituted γ-butyrolactones, optionally alkyl substituted δ-valerolactones, optionally alkyl substituted ε-caprolactones.

15. The process according to any of claims 9 to 14, wherein further contains an aprotic solvent in step a).
